# EUROPEAN PATENT APPLICATION

(11) **EP 3 861 989 A1**
(43) Date of publication of application: **11.08.2021**
(21) Application number: 20156003.4
(22) Date of filing: 07.02.2020
(51) Int. Cl.: A61K 9/14, A61K 9/20, A61K 9/28, A61K 31/44

(54) **PHARMACEUTICAL COMPOSITION CONTAINING REGORAFENIB AND A STABILIZING AGENT**

(71) Applicant: Bayer Aktiengesellschaft, 51373 Leverkusen (DE)
(72) Inventor: The designation of the inventor has not yet been filed
(74) Representative: BIP Patents

(57) **Abstract**

The present invention relates to an enteric coated pharmaceutical composition comprising a solid dispersion comprising regorafenib and at least one stabilizing agent outside of the solid dispersion, its process of preparation and its use for treating disorders.

## Description

The present invention relates to an enteric coated pharmaceutical composition comprising a solid dispersion comprising regorafenib and at least one stabilizing agent outside of the solid dispersion, its process of preparation and its use for treating disorders.

Regorafenib which is 4{4-[3-(4-chloro-3-trifluoromethylphenyl)-ureido]-3-fluorophenoxy}-pyridine-2-carboxylic acid methylamide, a compound of formula (I) 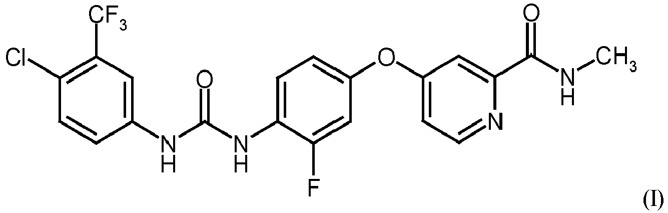 is a potent anti-cancer and anti-angiogenic agent that possesses various activities including inhibitory activity on the VEGFR, PDGFR, raf, p38, and/or flt-3 kinase signalling molecules and it can be used in treating various diseases and conditions like hyper-proliferative disorders such as cancers, tumors, lymphomas, sarcomas and leukemias as described in WO 2005/009961. Furthermore salts of the compound of formula (I) such as its hydrochloride, mesylate and phenylsulfonate are mentioned in WO 2005/009961. The monohydrate of the compound of formula (I) is mentioned in WO 2008/043446. An improved process for the manufacturing of regorafenib in high purity is described in WO 2011/128261. Due to the limited solubility of regorafenib monohydrate (see table 1) an applicable pharmaceutical composition containing regorafenib is in form of a solid dispersion as described in WO 2006/026500. WO 2014/039677 describes a solid dispersion containing regorafenib and which is coated with a polyvinylalcohol coating.

Today, the majority of new active pharmaceutical ingredients (APIs) shows the properties of poor solubility and subsequently reduced bioavailability (D. Alonzo: "Understanding the behavior of amorphous pharmaceutical systems during dissolution." Pharm. Res. 27, 608-618 (2010)). One approach to overcome these issues is embedding the amorphous API into water-soluble polymers forming an Amorphous Solid Dispersion (ASD). Once these systems get into contact with gastrointestinal media, dissolution will occur to a supersaturated state, which is more or less stabilized by the polymer. This so-called "spring and parachute" approach (C. Brough; "Amorphous solid dispersions and nanocrystal technologies for poorly water-soluble drug delivery." Int. J. Pharm. 453, 157-166 (2013)) has been shown to significantly enhance the bioavailability of poorly water-soluble APIs. One major challenge in administration of these APIs is the high inter-individual variability of drug performance (M. Herbrink: "Variability in bioavailability of small molecular tyrosine kinase inhibitors." Cancer Treat. Rev. 41, 412-422 (2015)). Another inherent issue of amorphous solid dispersions is the instability of the solid state which results in a tendency for recrystallisation of the drug and/or excipients during storage (Andrews et al., Journal of Pharmacy and Pharmacology; 62: 1580-1590 (2010)). This may be accompanied by a break-down of dissolution and bioavailability.The problem to be solved by the present invention is to provide a pharmaceutical composition containing regorafenib which shows stable dissolution and high bioavailability wherein the variability of the bioavailability is decreased.

Surprisingly the pharmaceutical composition according to the invention shows a supersaturation stabilization of the active ingredient regorafenib for improved bioavailability.

The present invention pertains to a pharmaceutical composition comprising a solid dispersion comprising regorafenib - which is the compound of the formula (I) 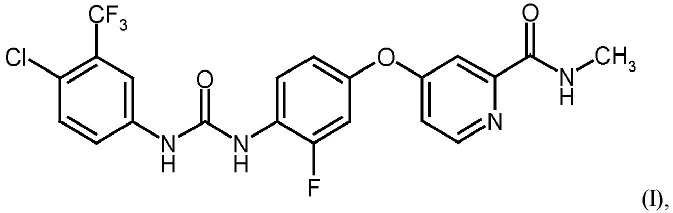 - and at least one pharmaceutically acceptable excipient inside of the solid dispersion, and at least one stabilizing agent wherein the stabilizing agent is outside of the solid dispersion and is preferably selected from the group consisting of methyl cellulose, ethyl cellulose, hydroxyethyl methyl cellulose (HPMC), hydroxypropyl methyl cellulose, hydroxyethyl cellulose, hydroxypropyl cellulose and its acetate, succinate, proprionate, butyrate, adipate, suberate, sebacate and phthalate ester derivatives like carboxy methyl cellulose, cellulose acetate, cellulose acetate phthalate, hydroxypropyl methyl cellulose phthalate, hydroxypropyl methyl cellulose phthalate acetate succinate, hydroxypropyl methyl cellulose acetate succinate and carmellose sodium and mixtures thereof, in particular carmellose sodium, hydroxypropyl methyl cellulose acetate succinate (HPMCAS), hydroxypropyl methyl cellulose phthalate, hydroxypropyl methyl cellulose and mixtures thereof, and wherein the pharmaceutical composition is enteric coated.

Name and meaning of excipients used in the present description are in accordance with the US or European pharmacopoeia.

The at least one stabilizing agent can be present in the enteric coating or in the area between the enteric coating and the solid dispersion or it can be present in the enteric coating and the area between the coating and the solid dispersion. Preferably the stabilizing agent is present in the coating or in the area between the coating and the solid dispersion.

In a preferred embodiment the pharmaceutical composition according to the invention consists of a mixture of further excipients (blend) and the solid dispersion particles, altogether mixed and formulated into tablets which are finally coated by an enteric coating and wherein the at least one stabilizing agent is present in the blend and/or in the enteric coating, most preferably is not present in the solid dispersion particles.

According to the present invention the blend is the part of the pharmaceutical composition which is neither the solid dispersion nor the coating.

The pharmaceutical composition according to the present invention can be utilized to achieve the desired pharmacological effect by administration to a patient in need thereof. A patient, for the purpose of this invention, is a mammal, including a human, in need of treatment for the particular condition or disease. Therefore, the present invention includes pharmaceutical compositions which are comprised of a pharmaceutically acceptable excipient and a pharmaceutically effective amount of a compound of the invention. A pharmaceutically acceptable excipient is any excipient which is relatively non-toxic and innocuous to a patient at concentrations consistent with effective activity of the active ingredient so that any side effects ascribable to the carrier do not vitiate the beneficial effects of the active ingredient. A pharmaceutically effective amount of compound is that amount which produces a result or exerts an influence on the particular condition being treated.

The term "the compound of formula (I)" or "regorafenib" refer to 4-{4-[({[4-chloro-3-(trifluoromethyl)phenyl]amino}carbonyl)amino]-3-fluorophenoxy}-*N*-methylpyridine-2-carboxamide as depicted in formula (I).

The term "compound of the invention" or "active agent" or "active ingredient" refer to regorafenib.

The total amount of the active ingredient (compound of the invention) to be administered preferably via the oral route using the pharmaceutical composition of the present invention will generally range from about 0.1 mg/kg to about 50 mg/kg body weight per day. Based upon standard laboratory techniques known to evaluate compounds useful for the treatment of hyper-proliferative disorders, by standard toxicity tests and by standard pharmacological assays for the determination of treatment of the conditions identified above in mammals, and by comparison of these results with the results of known medicaments that are used to treat these conditions, the effective dosage of the pharmaceutical compositions of this invention can readily be determined by those skilled in the art. The amount of the administered active ingredient can vary widely according to such considerations as the particular compound and dosage unit employed, the mode and time of administration, the period of treatment, the age, sex, and general condition of the patient treated, the nature and extent of the condition treated, the rate of drug metabolism and excretion, the potential drug combinations and drug-drug interactions, and the like.

Preference is given to an amount of regorafenib in the pharmaceutical composition from 4 to 400 mg, preferably from 10 to 200 mg, more preferably from 10 to 100 mg.

An aspect of the invention of particular interest is a pharmaceutical composition comprising regorafenib in an amount of 4 to 400 mg, preferably from 10 to 200 mg, more preferably from 10 to 100 mg. Most preferably the amount of regorafenib in the composition is 30, 40 or 60 mg.

The daily dose of the compound of the present invention, in particular regorafenib, is from 10 to 1000 mg, preferably 40 to 500 mg, more preferably 50 to 240 mg, e.g. 60, 90, 120 or 160 mg.

The pharmaceutical composition according to the invention is administered one or more, preferably up to three, more preferably up to two times per day. Preference is given to an administration via the oral route.

Nevertheless, it may in some cases be advantageous to deviate from the amounts specified, depending on body weight, individual behaviour toward the active ingredient, type of preparation and time or interval over which the administration is affected. For instance, less than the aforementioned minimum amounts may be sufficient in some cases, while the upper limit specified has to be exceeded in other cases. In the case of administration of relatively large amounts, it may be advisable to divide these into several individual doses over the day.

This pharmaceutical composition will be utilized to achieve the desired pharmacological effect by preferably oral administration to a patient in need thereof, and will have advantageous properties in terms of drug release, bioavailability, and/or compliance in mammals. A patient, for the purpose of this invention, is a mammal, including a human, in need of treatment for the particular condition or disease.

The pharmaceutical composition according to the invention is preferably a solid pharmaceutical compositions and is administered orally or rectally, preferably orally.

Pharmaceutical compositions according to the invention include but are not limited to granules, pellets, tablets, dragées, pills, melts or solid dispersions and may be prepared according to methods known to the art for the manufacture of pharmaceutical compositions. Preference is given to tablets, solid dispersions, pellets and granules. Most preferably the pharmaceutically compositions according to the invention is a tablet.

Preference is given to a enteric coated pharmaceutical composition which is a an delayed-release tablet, more preferably a gastro-resistant tablet according to European Pharmacopoeia 9th Edition 2019(9.8).

Pharmaceutical compositions according to the invention is in the form of a solid dispersion or a pharmaceutical composition comprising a solid dispersion. The solid dispersion may be a solid solution, glass solution, glass suspension, amorphous precipitation in a crystalline carrier, eutectic or monotectic, compound or complex formation or combinations thereof.

A solid dispersion according to the present invention is a solid dispersion matrix which comprises at least the compound of the invention, preferably in amorphous state, and a pharmaceutically acceptable solid dispersion matrix agent.

The term "solid dispersion matrix agents" as used herein refers to both polymeric excipients, non-polymeric excipients and combinations thereof, capable of dissolving or dispersing the compound of the invention.

A solid dispersion matrix agent according to the present invention includes but is not limited to polyvinylpyrrolidone (PVP), vinylpyrrolidone/vinylacetate copolymer, polyalkylene glycol (i.e. polyethylene glycol), hydroxyalkyl cellulose (i.e. hydroxypropyl cellulose), hydroxyalkyl methyl cellulose (i.e. hydroxypropyl methyl cellulose and hydroxypropyl methyl cellulose acetate succinate), carboxymethyl cellulose, sodium carboxymethyl cellulose, ethyl cellulose, polymethacrylates, polyvinyl alcohol, polyvinyl acetate, vinyl alcohol/vinyl acetate copolymer, polyglycolized glycerides, xanthan gum, carrageenan, chitosan, chitin, polydextrin, dextrin, starch, proteins or a mixture thereof.

Another aspect of the invention is a pharmaceutical composition comprising a solid dispersion, wherein the solid dispersion matrix agent includes but is not limited to a sugar and/or sugar alcohol and/or cyclodextrin, for example sucrose, lactose, fructose, maltose, raffinose, sorbitol, lactitol, mannitol, maltitol, erythritol, inositol, trehalose, isomalt, inulin, maltodextrin, β-cyclodextrin, hydroxypropyl-β-cyclodextrin or sulfobutylether cyclodextrin or a mixture thereof.

In a preferred embodiment at least one from the group of polyvinylpyrrolidone, copovidone, polyethylene glycol and polyethylene oxide or a mixture thereof is used as solid dispersion matrix agent. Most preferably polyvinylpyrrolidone is used as solid dispersion matrix agent.

In an embodiment of particular interest the solid dispersion comprises the compound of the invention and the solid dispersion matrix agent in a weight ratio of 1:0.5 to 1:20, preferably 1:1 to 1:10, most preferably 1:1 to 1:5.

Additional suitable excipients that are useful in the formation of the pharmaceutical composition according to the present invention and which are present in the blend include, but are not limited to alcohols, organic acids, organic bases, amino acids, phospholipids, waxes, salts, fatty acid esters, polyoxyethylene sorbitan fatty acid esters, and urea.

The pharmaceutical composition according to the present invention may contain certain additional pharmaceutical acceptable ingredients in the blend, such as surfactants, fillers, disintegrants, recrystallization inhibitors, plasticizers, defoamers, antioxidants, detackifier, pH-modifiers, glidants and lubricants.

Another aspect of the invention of particular interest is a pharmaceutical composition according to the present invention containing croscarmellose sodium, sodium starch glycolate, crospovidone (crosslinked polyvinylpyrrolidone), low substituted hydroxypropyl cellulose (L-HPC), starch, microcrystalline cellulose or a combination thereof as carrier or disintegrant in the solid dispersion. Preferably the solid dispersion comprises microcrystalline cellulose and/or croscarmellose sodium.

In another preferred embodiment, the solid dispersion comprises polyvinylpyrrolidone, croscarmellose sodium and optionally microcrystalline cellulose.

An embodiment of particular interest the solid dispersion comprises the compound of the invention and the sum of carrier and disintegrant in a weight ratio of 1:0.5 to 1:20, preferably 1:1 to 1:10, most preferably 1:1 to 1:6.

Stabilizing agents of the present pharmaceutical composition are selected from the group consisting of methyl cellulose, ethyl cellulose, hydroxyethyl methyl cellulose, hydroxyethyl cellulose, hydroxypropyl methyl cellulose (HPMC), hydroxypropyl cellulose and its acetate, succinate, proprionate, butyrate, adipate, suberate, sebacate and phthalate ester derivatives like carboxy methyl cellulose, cellulose acetate, cellulose acetate phthalate, hydroxypropyl methyl cellulose phthalate, hydroxypropyl methyl cellulose phthalate acetate succinate, hydroxypropyl methyl cellulose acetate succinate and carmellose sodium and mixtures thereof. More preferably hydroxypropyl methyl cellulose (HPMC), hydroxypropyl methyl cellulose acetate succinate (HPMCAS), hydroxypropyl methyl cellulose phthalate, hydroxycarboxy methyl cellulose, hydroxymethyl cellulose, hydroxyethyl cellulose, hydroxypropyl cellulose, croscarmellose sodium or mixtures thereof is used as stabilizing agent. Most preferably hydroxypropyl methyl cellulose acetate succinate (HPMCAS), hydroxypropyl methyl cellulose (HPMC) or mixtures thereof is used as stabilizing agent.

Hydroxypropyl methyl cellulose acetate succinate (HPMCAS) is a polymer based on hydroxypropyl methyl cellulose (Hypromellose) wherein the hydroxyl groups of this backbone acetyl and succinoyl groups are introduced. By chemical substitution levels of these acetyl and succinoyl groups a pH range from 5.5 to 6.5 is obtained. Thus this polymer is insoluble in gastric juice and immediately dissolves when the enteric preparation transfers to the small intestine and can therefore be applied as an enteric coating agent.

The weight amount of the at least one stabilizing agent in the pharmaceutical composition outside of the solid dispersion is at least 2%, preferably at least 5%, more preferably at least 10%, most preferably at least 15% based on the total weight of regorafenib in the pharmaceutical composition. For clarification purposes: Even if the stabilizing agent is present inside the solid dispersion (e.g. as matrix agent) and outside the solid dispersion, only the amount outside of the solid dispersion is to be considered in the beforementioned calculation. The area outside the solid dispersion includes any coating. In case more than one stabilizing agent is present in the pharmaceutical composition outside of the solid dispersion the values mentioned relate to the sum of the single amounts of all such stabilizing agents.

The weight amount of the at least one stabilizing agent in the pharmaceutical composition outside of the solid dispersion can be up to 400% (or in some cases even more) based on the total weight of regorafenib in the pharmaceutical composition

Coatings are an essential part in the formulation of pharmaceutical dosage form to achieve superior aesthetic quality (e.g., color, texture, mouth feel, and taste masking), physical and chemical protection for the drugs in the dosage forms, a reduced porosity of the tablet core and modification of drug release characteristics. Certain solid dosage forms are desired to be disintegrated only when they arrive at the intestinal canals upon oral administration and they are imparted with entero-soluble property by providing so-called enteric coating on the surface. Enteric coatings are resistant to stomach acid for required periods of time depending on the composition and/or thickness thereof, before they begin to disintegrate and allow for slow release of drug in the stomach and/or upper intestines.

Enteric coatings of the present pharmaceutical composition include but are not limited to waxes, shellac, sodium alginate, dextrins, zein, amylose starch and its derivatives, polyvinylacetate and its derivatives such as polyvinylacetate phthalate, neutral copolymer of polymethacrylic acid esters (Eudragits) and copolymers of methacrylic acid and methacrylic acid methyl ester (Eudragits), cellulose acetate trimellitate, cellulose acetate phthalate, cellulose acetate butyrate, cellulose acetate succinate, hydroxypropyl methyl cellulose phthalate, hydroxypropyl methyl cellulose acetate succinate or mixtures thereof. Preferably the enteric coating is selected from the group consisting of cellulose acetate trimellitate, cellulose acetate phthalate, cellulose acetate butyrate, cellulose acetate succinate, hydroxypropyl methyl cellulose phthalate, hydroxypropyl methyl cellulose acetate succinate or mixtures thereof. More preferably the enteric coating of the pharmaceutical composition of the present invention is selected from group consisting of hydroxypropyl methyl cellulose acetate succinate and/or hydroxypropyl methyl cellulose phthalate. Most preferably hydroxypropyl methyl cellulose acetate succinate is used as enteric coating agent.

In a preferred aspect of the present invention the stabilizing agent is selected from the group comprising hydroxypropyl methyl cellulose phthalate, hydroxypropyl methyl cellulose acetate succinate or mixtures thereof, most preferably hydroxypropyl methyl cellulose acetate succinate and forms the enteric coating of the pharmaceutical composition.

In another preferred embodiment the at least one stabilizing agent is present only in the enteric coating.

Most available enteric coating polymers begin to become soluble at pH 5.5 and above, with maximum solubility rates at pH values greater than 6.5.

The enteric coating may being combined with a further coating wherein preferably the further coating is below the enteric coating layer. The further coating of the pharmaceutical composition of the present invention can comprise a polyvinyl alcohol based polymer as film-forming agent. The polyvinyl alcohol based polymer includes but is not limited to fully hydrolysed polyvinyl alcohol polymer, partially hydrolysed polyvinyl alcohol polymer (contains free alcohol groups and esterified alcohol groups i.e. as acetate) esterified polyvinyl alcohol polymer for example polyvinyl acetate polymer, a co-polymer of the aforementioned with polyethylene glycol for example a polyvinyl alcohol-polyethylene glycol co-polymer or a mixture of the aforementioned. Preference is given to a partially hydrolysed polyvinyl alcohol polymer as further coating.

The polyvinyl alcohol based polymer in the further coating is present in an amount of 30 to 70%, preferably 35 to 60%, more preferably 35 to 50% by weight of the total coating.

In a preferred embodiment the pharmaceutical composition according to the present invention comprises a solid dispersion comprising regorafenib and at least one pharmaceutically acceptable excipient inside of the solid dispersion, and at least one stabilizing agent outside of the solid dispersion, most preferably HPMCAS, which forms the enteric coating, and the composition is coated in addition by a polyvinyl coating between the enteric coating and the remaining composition. More preferably the stabilizing agent is only part of the enteric coating and is not present in the remaining composition.

Furthermore the enteric and/or the further coating of the pharmaceutical composition of the present invention comprises optionally one or more further pharmaceutically acceptable excipients such as plasticizers, colorants, opacifiers, anti-tacking agents, dispersing agents and suspending agents.

Plasticizers which may be used in the coating include but are not limited to polyethylene glycol, propylene glycol, sorbitol, glycerol, maltitol, xylitol, mannitol, erythritol, glycerol trioleate, tributyl citrate, triethyl citrate acetyl triethyl citrate, glyceryl triacetate, stearic acid, medium chain triglycerides or a mixture thereof. Preference is given to polyethylene glycol, medium chain triglycerides and/or stearic acid.

The plasticizer in the coating may be present in an amount of 5 to 30%, preferably 8 to 25%, more preferably 10 to 20% by weight of the total coating.

Colorants which may be used in the coating include but are not limited to ferric oxide red, ferric oxide yellow, ferric oxide black, titanium dioxide, indigotine, sunset yellow FCF, tartrazin, erythrosine, quinoline yellow, carbon black, anthocyanin, riboflavin, carmine, curcumin, chlorophyll, carotene or a mixture thereof. Preference is given to ferric oxides and titanium dioxide.

The colorants in sum in the coating are present in an amount of 5 to 40%, preferably 8 to 30%, more preferably 10 to 20% by weight of the total coating.

Anti-tacking agents which may be used in the coating include but are not limited to talc, magnesium stearate, stearic acid, lecithin, soy lecithin, mineral oil, carnauba wax, acetylated monoglycerides, polysorbate or a mixture thereof. Preference is given to talc, lecithin, soy lecithin, and polysorbate.

Anti-tacking agents in sum in the coating are present in an amount of 3 to 30%, preferably 5 to 25%, more preferably 10 to 20% by weight of the total coating.

Opacifiers which may be used in the coating include by are not limited to talc and titanium dioxide. Opacifiers in sum in the coating are present in an amount of 10 to 45%, preferably 15 to 35%, more preferably 15 to 25% by weight of the total coating.

An aspect of the invention of particular interest is a composition, wherein the solid dispersion is substantially homogeneous.

An aspect of the invention of particular interest is a pharmaceutical composition, in which the compound of the invention is amorphous. That means, in the final formulation, the active pharmaceutical ingredient may be molecularly dispersed in the excipient matrix or may be dispersed as fine nano-crystalline or amorphous particles which form during solvent evaporation or cooling of the melt. By that the solid dispersion matrix polymers can be very effective solid-state stabilizers.

The present invention achieves a stable formulation with maximum bioavailability, stabilization of the amorphous drug through its shelf life and maintaining supersaturation of the drug in solution in vivo after administration.

One embodiment of the present invention is pharmaceutical composition comprising a solid dispersion comprising regorafenib and at least one pharmaceutically acceptable excipient inside of the solid dispersion, and at least one stabilizing agent outside of the solid dispersion which is selected from the group consisting of methyl cellulose, ethyl cellulose, hydroxyethyl methyl cellulose, hydroxyethyl cellulose, hydroxypropyl methyl cellulose (HPMC), hydroxypropyl cellulose and its acetate, succinate, proprionate, butyrate, adipate, suberate, sebacate and phthalate ester derivatives like carboxy methyl cellulose, cellulose acetate, cellulose acetate phthalate, hydroxypropyl methyl cellulose phthalate, hydroxypropyl methyl cellulose phthalate acetate succinate, hydroxypropyl methyl cellulose acetate succinate and carmellose sodium and mixtures thereof wherein the pharmaceutical composition is enteric coated.

Preference is given to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of hydroxypropyl methyl cellulose (HPMC), hydroxypropyl methyl cellulose acetate succinate (HPMCAS), hydroxypropyl methyl cellulose phthalate, hydroxycarboxy methyl cellulose, hydroxymethyl cellulose, hydroxyethyl cellulose, hydroxypropyl cellulose, croscarmellose sodium and mixtures thereof wherein the pharmaceutical composition is enteric coated.

Also preference is given to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of hydroxypropyl methyl cellulose (HPMC), hydroxypropyl methyl cellulose acetate succinate, hydroxypropyl methyl cellulose phthalate, hydroxycarboxy methyl cellulose, hydroxymethyl cellulose, hydroxyethyl cellulose, hydroxypropyl cellulose, croscarmellose sodium and mixtures thereof wherein the pharmaceutical composition is enteric coated and the enteric coating comprises cellulose acetate trimellitate, cellulose acetate phthalate, cellulose acetate butyrate, cellulose acetate succinate, hydroxypropyl methyl cellulose phthalate, hydroxypropyl methyl cellulose acetate succinate or mixtures thereof.

More preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of carmellose sodium, hydroxypropyl methyl cellulose acetate succinate (HPMCAS), hydroxypropyl methyl cellulose phthalate, hydroxypropyl methyl cellulose (HPMC), hydroxypropyl cellulose, hydroxyethyl cellulose or mixtures thereof wherein the pharmaceutical composition is enteric coated.

Also more preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of carmellose sodium, hydroxypropyl methyl cellulose acetate succinate (HPMCAS), hydroxypropyl methyl cellulose phthalate, hydroxypropyl methyl cellulose (HPMC), hydroxypropyl cellulose, hydroxyethyl cellulose or mixtures thereof wherein the pharmaceutical composition is enteric coated and the enteric coating comprises hydroxypropyl methyl cellulose acetate succinate and/or hydroxypropyl methyl cellulose phthalate.

Also more preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of hydroxypropyl methyl cellulose acetate succinate and/or hydroxypropyl methyl cellulose phthalate wherein the stabilizing agent is only present inside the enteric coating.

Most preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is hydroxypropyl methyl cellulose acetate succinate (HPMCAS) hydroxypropyl methyl cellulose (HPMC) or mixtures thereof wherein the pharmaceutical composition is enteric coated.

Also most preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is hydroxypropyl methyl cellulose acetate succinate (HPMCAS) hydroxypropyl methyl cellulose (HPMC) or mixtures thereof wherein the pharmaceutical composition is enteric coated and the enteric coating comprises hydroxypropyl methyl cellulose acetate succinate.

Also most preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is hydroxypropyl methyl cellulose acetate succinate wherein the pharmaceutical composition is enteric coated and hydroxypropyl methyl cellulose acetate succinate is only present inside the enteric coating.

One embodiment of the present invention is pharmaceutical composition comprising a solid dispersion comprising regorafenib and at least one pharmaceutically acceptable excipient inside of the solid dispersion, and at least one stabilizing agent outside of the solid dispersion which is selected from the group consisting of methyl cellulose, ethyl cellulose, hydroxyethyl methyl cellulose, hydroxyethyl cellulose, hydroxypropyl methyl cellulose (HPMC), hydroxypropyl cellulose and its acetate, succinate, proprionate, butyrate, adipate, suberate, sebacate and phthalate ester derivatives like carboxy methyl cellulose, cellulose acetate, cellulose acetate phthalate, hydroxypropyl methyl cellulose phthalate, hydroxypropyl methyl cellulose phthalate acetate succinate, hydroxypropyl methyl cellulose acetate succinate and carmellose sodium and mixtures thereof wherein the pharmaceutical composition is enteric coated and below the enteric coating layer is a further coating layer.

Preference is given to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of hydroxypropyl methyl cellulose (HPMC), hydroxypropyl methyl cellulose acetate succinate, hydroxypropyl methyl cellulose phthalate, hydroxycarboxy methyl cellulose, hydroxymethyl cellulose, hydroxyethyl cellulose, hydroxypropyl cellulose, croscarmellose sodium and mixtures thereof wherein the pharmaceutical composition is enteric coated and below the enteric coating layer is a further coating layer.

Also preference is given to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of hydroxypropyl methyl cellulose (HPMC), hydroxypropyl methyl cellulose acetate succinate, hydroxypropyl methyl cellulose phthalate, hydroxycarboxy methylcellulose, hydroxymethyl cellulose, hydroxyethyl cellulose, hydroxypropyl cellulose, croscarmellose sodium and mixtures thereof wherein the pharmaceutical composition is enteric coated and the enteric coating comprises cellulose acetate trimellitate, cellulose acetate phthalate, cellulose acetate butyrate, cellulose acetate succinate, hydroxypropyl methyl cellulose phthalate, hydroxypropyl methyl cellulose acetate succinate or mixtures thereof.

More preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of carmellose sodium, hydroxypropyl methyl cellulose acetate succinate (HPMCAS), hydroxypropyl methyl cellulose phthalate, hydroxypropyl methyl cellulose (HPMC), hydroxypropyl cellulose, hydroxyethyl cellulose or mixtures thereof wherein the pharmaceutical composition is enteric coated and below the enteric coating layer is a further coating layer which comprises a polyvinylalcohol.

Also more preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of carmellose sodium, hydroxypropyl methyl cellulose acetate succinate (HPMCAS), hydroxypropyl methyl cellulose phthalate, hydroxypropyl methyl cellulose (HPMC), hydroxypropyl cellulose, hydroxyethyl cellulose or mixtures thereof wherein the pharmaceutical composition is enteric coated and the enteric coating comprises hydroxypropyl methyl cellulose acetate succinate and/or hydroxypropyl methyl cellulose phthalate.

Also more preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of hydroxypropyl methyl cellulose acetate succinate and/or hydroxypropyl methyl cellulose phthalate wherein the stabilizing agent is only present inside the enteric coating.

Most preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is hydroxypropyl methyl cellulose acetate succinate (HPMCAS) hydroxypropyl methyl cellulose (HPMC) or mixtures thereof wherein the pharmaceutical composition is enteric coated and below the enteric coating layer is a further coating layer which comprises a polyvinylalcohol.

Also most preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is hydroxypropyl methyl cellulose acetate succinate (HPMCAS) hydroxypropyl methyl cellulose (HPMC) or mixtures thereof wherein the pharmaceutical composition is enteric coated and the enteric coating comprises hydroxypropyl methyl cellulose acetate succinate and below the enteric coating layer is a further coating layer which comprises a polyvinylalcohol.

Also most preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is hydroxypropyl methyl celluloseacetate succinate wherein the pharmaceutical composition is enteric coated and hydroxypropyl methyl cellulose acetate succinate is only present inside the enteric coating.

One embodiment of the present invention is pharmaceutical composition comprising a solid dispersion comprising regorafenib and at least one pharmaceutically acceptable excipient inside of the solid dispersion, and at least one stabilizing agent outside of the solid dispersion which is selected from the group consisting of methyl cellulose, ethyl cellulose, hydroxyethyl methyl cellulose, hydroxyethyl cellulose, hydroxypropyl methyl cellulose (HPMC), hydroxypropyl cellulose and its acetate, succinate, proprionate, butyrate, adipate, suberate, sebacate and phthalate ester derivatives like carboxy methyl cellulose, cellulose acetate, cellulose acetate phthalate, hydroxypropyl methyl cellulose phthalate, hydroxypropyl methyl cellulose phthalate acetate succinate, hydroxypropyl methyl cellulose acetate succinate and carmellose sodium and mixtures thereof wherein the pharmaceutical composition is enteric coated wherein the solid dispersion comprises a solid dispersion matrix agent selected from the list consisting of polyvinylpyrrolidone (PVP), vinylpyrrolidone/vinylacetate copolymer, hydroxypropyl cellulose, hydroxypropyl methyl cellulose, hydroxypropyl methyl cellulose acetate succinate, carboxymethyl cellulose, sodium carboxymethyl cellulose, ethyl cellulose, polymethacrylates, polyvinyl alcohol, polyvinyl acetate, vinyl alcohol/vinyl acetate copolymer, polyglycolized glycerides, xanthan gum, carrageenan, chitosan, chitin, polydextrin, dextrin, starch, proteins or a mixture thereof.

Preference is given to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of hydroxypropyl methyl cellulose (HPMC), hydroxypropyl methyl cellulose acetate succinate, hydroxypropyl methyl cellulose phthalate, hydroxycarboxy methyl cellulose, hydroxymethyl cellulose, hydroxyethyl cellulose, hydroxypropyl cellulose, croscarmellose sodium and mixtures thereof wherein the pharmaceutical composition is enteric coated and wherein the solid dispersion comprises a solid dispersion matrix agent selected from the list consisting of polyvinylpyrrolidone, copovidone, polyethylene glycol, polyethylene oxide or a mixture thereof.

Also preference is given to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of hydroxypropyl methyl cellulose (HPMC), hydroxypropyl methyl cellulose acetate succinate, hydroxypropyl methyl cellulose phthalate, hydroxycarboxy methyl cellulose, hydroxymethyl cellulose, hydroxyethyl cellulose, hydroxypropyl cellulose, croscarmellose sodium and mixtures thereof wherein the pharmaceutical composition is enteric coated and the enteric coating comprises cellulose acetate trimellitate, cellulose acetate phthalate, cellulose acetate butyrate, cellulose acetate succinate, hydroxypropyl methyl cellulose phthalate, hydroxypropyl methyl cellulose acetate succinate or mixtures thereofwherein the solid dispersion comprises a solid dispersion matrix agent selected from the list consisting of polyvinylpyrrolidone, copovidone, polyethylene glycol, polyethylene oxide or a mixture thereof.

More preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of carmellose sodium, hydroxypropyl methyl cellulose acetate succinate (HPMCAS), hydroxypropyl methyl cellulose phthalate, hydroxypropyl methyl cellulose (HPMC), hydroxypropyl cellulose, hydroxyethyl cellulose or mixtures thereof wherein the pharmaceutical composition is enteric coated and wherein the solid dispersion comprises a solid dispersion matrix agent selected from the list consisting of polyvinylpyrrolidone, copovidone, polyethylene glycol, polyethylene oxide or a mixture thereof.

Also more preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of carmellose sodium, hydroxypropyl methyl cellulose acetate succinate (HPMCAS), hydroxypropyl methyl cellulose phthalate, hydroxypropyl methyl cellulose (HPMC), hydroxypropyl cellulose, hydroxyethyl cellulose or mixtures thereof wherein the pharmaceutical composition is enteric coated and the enteric coating comprises hydroxypropyl methyl cellulose acetate succinate and/or hydroxypropyl methyl cellulose phthalate and wherein the solid dispersion comprises a solid dispersion matrix agent selected from the list consisting of polyvinylpyrrolidone, copovidone, polyethylene glycol, polyethylene oxide or a mixture thereof.

Also more preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of hydroxypropyl methyl cellulose acetate succinate and/or hydroxypropyl methyl cellulose phthalate wherein the stabilizing agent is only present inside the enteric coating and wherein the solid dispersion comprises a solid dispersion matrix agent selected from the list consisting of polyvinylpyrrolidone, copovidone, polyethylene glycol, polyethylene oxide or a mixture thereof.

Most preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is hydroxypropyl methyl cellulose acetate succinate (HPMCAS) hydroxypropyl methyl cellulose (HPMC) or mixtures thereof wherein the pharmaceutical composition is enteric coated and wherein the solid dispersion comprises polyvinylpyrrolidone as solid dispersion matrix agent.

Also most preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is hydroxypropyl methyl cellulose acetate succinate (HPMCAS) hydroxypropyl methyl cellulose (HPMC) or mixtures thereof wherein the pharmaceutical composition is enteric coated and the enteric coating comprises hydroxypropyl methyl cellulose acetate succinate and wherein the solid dispersion comprises polyvinylpyrrolidone as solid dispersion matrix agent.

Also most preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is hydroxypropyl methyl cellulose acetate succinate wherein the pharmaceutical composition is enteric coated and hydroxypropyl methyl cellulose acetate succinate is only present inside the enteric coating and wherein the solid dispersion comprises polyvinylpyrrolidone as solid dispersion matrix agent.

One embodiment of the present invention is pharmaceutical composition comprising a solid dispersion comprising regorafenib and at least one pharmaceutically acceptable excipient inside of the solid dispersion, and at least one stabilizing agent outside of the solid dispersion which is selected from the group consisting of methyl cellulose, ethyl cellulose, hydroxyethyl methyl cellulose, hydroxyethyl cellulose, hydroxypropyl methyl cellulose (HPMC), hydroxypropyl cellulose and its acetate, succinate, proprionate, butyrate, adipate, suberate, sebacate and phthalate ester derivatives like carboxy methyl cellulose, cellulose acetate, cellulose acetate phthalate, hydroxypropyl methyl cellulose phthalate, hydroxypropyl methyl cellulose phthalate acetate succinate, hydroxypropyl methyl cellulose acetate succinate and carmellose sodium and mixtures thereof wherein the pharmaceutical composition is enteric coated and below the enteric coating layer is a further coating layer and wherein the solid dispersion comprises a solid dispersion matrix agent selected from the list consisting of polyvinylpyrrolidone (PVP), vinylpyrrolidone/vinylacetate copolymer, hydroxypropyl cellulose, hydroxypropyl methyl cellulose, hydroxypropyl methyl cellulose acetate succinate, carboxymethyl cellulose, sodium carboxymethyl cellulose, ethyl cellulose, polymethacrylates, polyvinyl alcohol, polyvinyl acetate, vinyl alcohol/vinyl acetate copolymer, polyglycolized glycerides, xanthan gum, carrageenan, chitosan, chitin, polydextrin, dextrin, starch, proteins or a mixture thereof.

Preference is given to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of hydroxypropyl methyl cellulose (HPMC), hydroxypropyl methyl cellulose acetate succinate, hydroxypropyl methyl cellulose phthalate, hydroxycarboxy methyl cellulose, hydroxymethyl cellulose, hydroxyethyl cellulose, hydroxypropyl cellulose, croscarmellose sodium and mixtures thereof wherein the pharmaceutical composition is enteric coated and below the enteric coating layer is a further coating layer and wherein the solid dispersion comprises a solid dispersion matrix agent selected from the list consisting of polyvinylpyrrolidone, copovidone, polyethylene glycol, polyethylene oxide or a mixture thereof.

Also preference is given to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of hydroxypropyl methyl cellulose (HPMC), hydroxypropyl methyl cellulose acetate succinate, hydroxypropyl methyl cellulose phthalate, hydroxycarboxy methyl cellulose, hydroxymethyl cellulose, hydroxyethyl cellulose, hydroxypropyl cellulose, croscarmellose sodium and mixtures thereof wherein the pharmaceutical composition is enteric coated and the enteric coating comprises cellulose acetate trimellitate, cellulose acetate phthalate, cellulose acetate butyrate, cellulose acetate succinate, hydroxypropyl methyl cellulose phthalate, hydroxypropyl methyl cellulose acetate succinate or mixtures thereof and wherein the solid dispersion comprises a solid dispersion matrix agent selected from the list consisting of polyvinylpyrrolidone, copovidone, polyethylene glycol, polyethylene oxide or a mixture thereof.

More preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of carmellose sodium, hydroxypropyl methyl cellulose acetate succinate (HPMCAS), hydroxypropyl methyl cellulose phthalate, hydroxypropyl methyl cellulose (HPMC), hydroxypropyl cellulose, hydroxyethyl cellulose or mixtures thereof wherein the pharmaceutical composition is enteric coated and below the enteric coating layer is a further coating layer which comprises a polyvinylalcohol and wherein the solid dispersion comprises a solid dispersion matrix agent selected from the list consisting of polyvinylpyrrolidone, copovidone, polyethylene glycol, polyethylene oxide or a mixture thereof.

Also more preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of carmellose sodium, hydroxypropyl methyl cellulose acetate succinate (HPMCAS), hydroxypropyl methyl cellulose phthalate, hydroxypropyl methyl cellulose (HPMC), hydroxypropyl cellulose, hydroxyethyl cellulose or mixtures thereof wherein the pharmaceutical composition is enteric coated and the enteric coating comprises hydroxypropyl methyl cellulose acetate succinate and/or hydroxypropyl methyl cellulose phthalate and wherein the solid dispersion comprises a solid dispersion matrix agent selected from the list consisting of polyvinylpyrrolidone, copovidone, polyethylene glycol, polyethylene oxide or a mixture thereof.

Also more preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of hydroxypropyl methyl cellulose acetate succinate and/or hydroxypropyl methyl cellulose phthalate wherein the stabilizing agent is only present inside the enteric coating and wherein the solid dispersion comprises a solid dispersion matrix agent selected from the list consisting of polyvinylpyrrolidone, copovidone, polyethylene glycol, polyethylene oxide or a mixture thereof.

Most preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is hydroxypropyl methyl cellulose acetate succinate (HPMCAS) hydroxypropyl methyl cellulose (HPMC) or mixtures thereof wherein the pharmaceutical composition is enteric coated and below the enteric coating layer is a further coating layer which comprises a polyvinylalcohol and wherein the solid dispersion comprises polyvinylpyrrolidone as solid dispersion matrix agent.

Also most preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is hydroxypropyl methyl cellulose acetate succinate (HPMCAS) hydroxypropyl methyl cellulose (HPMC) or mixtures thereof wherein the pharmaceutical composition is enteric coated and the enteric coating comprises hydroxypropyl methyl cellulose acetate succinate and below the enteric coating layer is a further coating layer which comprises a polyvinylalcohol and wherein the solid dispersion comprises polyvinylpyrrolidone as solid dispersion matrix agent.

Also most preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is hydroxypropyl methyl cellulose acetate succinate wherein the pharmaceutical composition is enteric coated and hydroxypropyl methyl cellulose acetate succinate is only present inside the enteric coating and wherein the solid dispersion comprises polyvinylpyrrolidone as solid dispersion matrix agent.

One embodiment of the present invention is pharmaceutical composition comprising a solid dispersion comprising regorafenib and at least one pharmaceutically acceptable excipient inside of the solid dispersion, and at least one stabilizing agent outside of the solid dispersion which is selected from the group consisting of methyl cellulose, ethyl cellulose, hydroxyethyl methyl cellulose, hydroxyethyl cellulose, hydroxypropyl methyl cellulose (HPMC), hydroxypropyl cellulose and its acetate, succinate, proprionate, butyrate, adipate, suberate, sebacate and phthalate ester derivatives like carboxy methyl cellulose, cellulose acetate, cellulose acetate phthalate, hydroxypropyl methyl cellulose phthalate, hydroxypropyl methyl cellulose phthalate acetate succinate, hydroxypropyl methyl cellulose acetate succinate and carmellose sodium and mixtures thereof wherein the pharmaceutical composition is enteric coated and the weight amount of the at least one stabilizing agent in the pharmaceutical composition outside of the solid dispersion is at least 2%, preferably at least 5%, more preferably at least 10%, most preferably at least 15% based on the total weight of regorafenib in the pharmaceutical composition.

Preference is given to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of hydroxypropyl methyl cellulose (HPMC), hydroxypropyl methyl cellulose acetate succinate, hydroxypropyl methyl cellulose phthalate, hydroxycarboxy methyl cellulose, hydroxymethyl cellulose, hydroxyethyl cellulose, hydroxypropyl cellulose, croscarmellose sodium and mixtures thereof wherein the pharmaceutical composition is enteric coated and the weight amount of the at least one stabilizing agent in the pharmaceutical composition outside of the solid dispersion is at least 2%, preferably at least 5%, more preferably at least 10%, most preferably at least 15% based on the total weight of regorafenib in the pharmaceutical composition.

Also preference is given to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of hydroxypropyl methyl cellulose (HPMC), hydroxypropyl methyl cellulose acetate succinate, hydroxypropyl methyl cellulose phthalate, hydroxycarboxy methyl cellulose, hydroxymethyl cellulose, hydroxyethyl cellulose, hydroxypropyl cellulose, croscarmellose sodium and mixtures thereof wherein the pharmaceutical composition is enteric coated and the enteric coating comprises cellulose acetate trimellitate, cellulose acetate phthalate, cellulose acetate butyrate, cellulose acetate succinate, hydroxypropyl methyl cellulose phthalate, hydroxypropyl methyl cellulose acetate succinate or mixtures thereof and the weight amount of the at least one stabilizing agent in the pharmaceutical composition outside of the solid dispersion is at least 2%, preferably at least 5%, more preferably at least 10%, most preferably at least 15% based on the total weight of regorafenib in the pharmaceutical composition.

More preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of carmellose sodium, hydroxypropyl methyl cellulose acetate succinate (HPMCAS), hydroxypropyl methyl cellulose phthalate, hydroxypropyl methyl cellulose (HPMC), hydroxypropyl cellulose, hydroxyethyl cellulose or mixtures thereof wherein the pharmaceutical composition is enteric coated and the weight amount of the at least one stabilizing agent in the pharmaceutical composition outside of the solid dispersion is at least 2%, preferably at least 5%, more preferably at least 10%, most preferably at least 15% based on the total weight of regorafenib in the pharmaceutical composition.

Also more preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of carmellose sodium, hydroxypropyl methyl cellulose acetate succinate (HPMCAS), hydroxypropyl methyl cellulose phthalate, hydroxypropyl methyl cellulose (HPMC), hydroxypropyl cellulose, hydroxyethyl cellulose or mixtures thereof wherein the pharmaceutical composition is enteric coated and the enteric coating comprises hydroxypropyl methyl cellulose acetate succinate and/or hydroxypropyl methyl cellulose phthalate and the weight amount of the at least one stabilizing agent in the pharmaceutical composition outside of the solid dispersion is at least 2%, preferably at least 5%, more preferably at least 10%, most preferably at least 15% based on the total weight of regorafenib in the pharmaceutical composition.

Also more preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is selected from the group consisting of hydroxypropyl methyl cellulose acetate succinate and/or hydroxypropyl methyl cellulose phthalate wherein the stabilizing agent is only present inside the enteric coating and the weight amount of the at least one stabilizing agent in the pharmaceutical composition outside of the solid dispersion is at least 2%, preferably at least 5%, more preferably at least 10%, most preferably at least 15% based on the total weight of regorafenib in the pharmaceutical composition.

Most preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is hydroxypropyl methyl cellulose acetate succinate (HPMCAS) hydroxypropyl methyl cellulose (HPMC) or mixtures thereof wherein the pharmaceutical composition is enteric coated and the weight amount of the at least one stabilizing agent in the pharmaceutical composition outside of the solid dispersion is at least 2%, preferably at least 5%, more preferably at least 10%, most preferably at least 15% based on the total weight of regorafenib in the pharmaceutical composition.

Also most preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is hydroxypropyl methyl cellulose acetate succinate (HPMCAS) hydroxypropyl methyl cellulose (HPMC) or mixtures thereof wherein the pharmaceutical composition is enteric coated and the enteric coating comprises hydroxypropyl methyl cellulose acetate succinate and the weight amount of the at least one stabilizing agent in the pharmaceutical composition outside of the solid dispersion is at least 2%, preferably at least 5%, more preferably at least 10%, most preferably at least 15% based on the total weight of regorafenib in the pharmaceutical composition.

Also most preferably the present invention relates to a pharmaceutical composition comprising a solid dispersion comprising regorafenib and at least one pharmaceutically acceptable excipient inside of the solid dispersion, and at least one stabilizing agent outside of the solid dispersion which is hydroxypropyl methyl cellulose acetate succinate wherein the pharmaceutical composition is enteric coated and hydroxypropyl methyl cellulose acetate succinate is only present inside the enteric coating and the weight amount of the at least one stabilizing agent in the pharmaceutical composition outside of the solid dispersion is at least 2%, preferably at least 5%, more preferably at least 10%, most preferably at least 15% based on the total weight of regorafenib in the pharmaceutical composition.

In this connection the pharmaceutical composition according to the invention - when investigated for release testing - contains 4-(4-amino-3-fluorophenoxy)pyridine-2-carboxylic acid methylamide (IUPAC: 4-(4-amino-3-fluorophenoxy)-N-methylpyridine-2-carboxamide) (AFP-PMA) in an amount of equal or less than 0.050%, that means from 0.001% to a maximum of 0.050%, preferably in an amount of equal or less than 0.025%, that means from 0.001% to a maximum of 0.025%, most preferably in an amount of equal or less than 0.015%, that means from 0.001% to a maximum of 0.015% by weight based on the amount of the compound of the formula (I). It is commonly understood that release testing is performed without undue delay after the manufacturing of a batch of the product has been completed. Release testing is also formally required before the respective product batch can be marketed.

Furthermore, the pharmaceutical composition according to the invention - when investigated at the end of the product shelf life - contains 4-(4-amino-3-fluorophenoxy)pyridine-2-carboxylic acid methylamide (IUPAC: 4-(4-amino-3-fluorophenoxy)-N-methylpyridine-2-carboxamide) (AFP-PMA) in an amount of equal or less than 0.10%, that means from 0.001% to a maximum of 0.10%, preferably in an amount of equal or less than 0.08%, that means from 0.001% to a maximum of 0.08%, most preferably in an amount of equal or less than 0.05%, that means from 0.001% to a maximum of 0.05% by weight based on the amount of the compound of the formula (I).

Another aspect of the present invention is a film-coated pharmaceutical composition, preferably a tablet, comprising regorafenib and - when investigated for release testing - 4-(4-amino-3-fluorophenoxy)pyridine-2-carboxylic acid methylamide (IUPAC: 4-(4-amino-3-fluorophenoxy)-N-methylpyridine-2-carboxamide) (AFP-PMA) in an amount of equal or less than 0.050%, that means from 0.001% to a maximum of 0.050%, preferably in an amount of equal or less than 0.025%, that means from 0.001% to a maximum of 0.025%, most preferably in an amount of equal or less than 0.015%, that means from 0.001% to a maximum of 0.015% by weight based on the amount of regorafenib and at least one pharmaceutically acceptable excipient.

Preference is given to a tablet comprising regorafenib and hydroxypropyl methyl cellulose acetate succinate (HPMC-AS) - when investigated for release testing -4-(4-amino-3-fluorophenoxy)pyridine-2-carboxylic acid methylamide (IUPAC: 4-(4-amino-3-fluorophenoxy)-N-methylpyridine-2-carboxamide) (AFP-PMA) in an amount of equal or less than 0.050%, that means from 0.001% to a maximum of 0.050%, preferably in an amount of equal or less than 0.025%, that means from 0.001% to a maximum of 0.025%, most preferably in an amount of equal or less than 0.015%, that means from 0.001% to a maximum of 0.015% by weight based on the amount of regorafenib and at least one pharmaceutically acceptable excipient wherein the tablet is coated by a coating comprising a polyvinyl alcohol based polymer in particular a partially hydrolysed polyvinyl alcohol polymer and optionally one or more further pharmaceutically acceptable excipients.

Still another aspect of the present invention is a film-coated pharmaceutical composition, preferably a tablet, comprising regorafenib and hydroxypropyl methyl cellulose acetate succinate - when investigated at the end of the product shelf life - 4-(4-amino-3-fluorophenoxy)pyridine-2-carboxylic acid methylamide (IUPAC: 4-(4-amino-3-fluorophenoxy)-N-methylpyridine-2-carboxamide) (AFP-PMA) in an amount of equal or less than 0.10%, that means from 0.001% to a maximum of 0.10%, preferably in an amount of equal or less than 0.08%, that means from 0.001% to a maximum of 0.08%, most preferably in an amount of equal or less than 0.05%, that means from 0.001% to a maximum of 0.05% by weight based on the amount of regorafenib and at least one pharmaceutically acceptable excipient.

Preference is given to a tablet comprising regorafenib and hydroxypropyl methyl cellulose acetate succinate - when investigated at the end of the product shelf life - 4-(4-amino-3-fluorophenoxy)pyridine-2-carboxylic acid methylamide (IUPAC: 4-(4-amino-3-fluorophenoxy)-N-methylpyridine-2-carboxamide) (AFP-PMA) in an amount of equal or less than 0.10%, that means from 0.001% to a maximum of 0.10%, preferably in an amount of equal or less than 0.08%, that means from 0.001% to a maximum of 0.08%, most preferably in an amount of equal or less than 0.05%, that means from 0.001% to a maximum of 0.05% by weight based on the amount of regorafenib and at least one pharmaceutically acceptable excipient wherein the tablet is coated by a coating comprising a polyvinyl alcohol based polymer in particular a partially hydrolysed polyvinyl alcohol polymer and optionally one or more further pharmaceutically acceptable excipients.

The pharmaceutical composition according to the invention is chemically stable for more than 18 months, preferably more than 24 months, most preferably more than 36 months during storage e.g. in climatic zones 1 to 2 , preferably in climatic zones 1 to 4b.

The pharmaceutical composition according to the invention is chemically stable and comprises 4-(4-amino-3-fluorophenoxy)pyridine-2-carboxylic acid methylamide (AFP-PMA) in an amount of or less than 0.100%, that means from 0.001% to a maximum of 0.100%, preferably in an amount of equal or less than 0.08%, that means from 0.001% to a maximum of 0.08%, most preferably in an amount of equal or less than 0.050%, that means from 0.001% to a maximum of 0.050% by weight based on the amount of regorafenib in the composition for at least 18 months, preferably at least 24 months, most preferably at least 36 months during storage e.g. in climatic zones 1 to 2, preferably in climatic zones 1 to 4b. Climatic zones are a well-known concept to define the storage conditions for long-term stability studies in order to determine the shelf-life of pharmaceutical products . For example, data obtained from storage at 25 °C and 60 % relative humidity are used to justify a shelf-life for climatic zones 1 to 2, whereas data obtained from storage at 40 °C and 75 % relative humidity are used to justify a shelf-life for climatic zones 1 to 4b.

The monthly increase rate of the amount of AFP-PMA in the pharmaceutical composition according to the invention during storage at 25°C / 60 % relative humidity is equal or less than 0.0015%, that means from 0.0001% to a maximum of 0.0015%, preferably equal or less than 0.001%, that means from 0.0001% to a maximum of 0.001% by weight based on the amount of regorafenib in the composition per month.

The monthly increase rate of the amount of AFP-PMA in the pharmaceutical composition according to the invention during storage at 30°C / 75 % relative humidity is equal or less than 0.0030%, that means from 0.0001% to a maximum of 0.0030%, preferably equal or less than 0.0025%, that means from 0.0001% to a maximum of 0.0025% by weight based on the amount of regorafenib in the composition per month.

### Process for manufacturing

The solid dispersion of the invention can be prepared according to methods known to the art for the manufacture of solid dispersions, such as fusion/melt technology, hot melt extrusion, solvent evaporation (i.e. freeze drying, spray drying or layering of powders of granules), coprecipitation, supercritical fluid technology and electrostatic spinning method which are for example described in WO 2006/026500.

Hot melt extrusion or solvent evaporation techniques are preferred processes for preparation of solid dispersion formulations of this invention.

A solvent suitable for manufacture of solid dispersions by solvent evaporation processes such as spray-drying, layering or fluid-bed granulation can be any compound, wherein the compound of the invention can be dissolved. Preferred solvents include alcohols (e.g. methanol, ethanol, n-propanol, isopropanol, and butanol), ketones (e.g. acetone, methyl ethyl ketone and methyl isobutyl ketone, butanone), esters (e.g. ethyl acetate and propyl acetate) and various other solvents such as acetonitrile, methylene chloride, chloroform, hexane, toluene, tetrahydrofurane, cyclic ethers, and 1,1,1-trichloroethane. Lower volatility solvents, such as dimethyl acetamide or dimethyl sulfoxide can also be used. Mixtures of solvents, such as 20% ethanol and 80% acetone, can also be used, as can mixtures with water as long as the drug and if necessary the matrix agent are sufficiently soluble to make the process practicable.

In a preferred embodiment the solvent used for manufacture of the solid dispersion is methanol, ethanol, n-propanol, isopropanol, acetone or a mixture thereof. More preferably a mixture of ethanol and acetone is used as solvent.

The pharmaceutical compositions according to the invention is coated according to methods known to the art as described e.g. in WO 2014/039677 like spraying the coating liquid in a pan or perforated drum coater onto the pharmaceutical composition.

The pharmaceutical compositions according to the invention can contain, but not necessarily, HPMC or HPMCAS as an excipient in the blend after granulation. These compositions will be coated with suitable excipients to achieve an enteric function of the coating.

Most film coatings are applied as aqueous or organic based or aqueous-organic based polymer solutions. Methods for applying enteric coating polymers are described in e.g. (Bauer et al., Wiss. Verl.-Ges., "Überzogene Arzneiformen", 1988, ISBN 3-8047-0812-9). There are also several solvent-free coating techniques being described or even applied such as compression coating, hot-melt coating, electrostatic spray powder coating, dry powder coating, supercritical fluid-based coating, and photocurable coating. (Pharm. Dev. Tech., 12, "Solventless Pharmaceutical Coating Processes: A Review", 2007, 115-131)

### Method for treatment:

The present invention also relates to a method for using the compound of the invention and compositions thereof, to treat mammalian hyper-proliferative disorders. This method comprises administering to a mammal in need thereof, including a human, an amount of a compound of the invention or composition thereof, which is effective to treat the disorder. Hyper-proliferative disorders include but are not limited to solid tumors, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukemias.

Examples of breast cancer include, but are not limited to invasive ductal carcinoma, invasive lobular carcinoma, ductal carcinoma in situ, and lobular carcinoma in situ.

Examples of cancers of the respiratory tract include, but are not limited to small-cell and non-small-cell lung carcinoma (NSCLC), as well as bronchial adenoma and pleuropulmonary blastoma.

Examples of brain cancers include, but are not limited to brain stem and hypophtalmic glioma, cerebellar and cerebral astrocytoma, medulloblastoma, ependymoma, as well as neuroectodermal and pineal tumor.

Tumors of the male reproductive organs include, but are not limited to prostate and testicular cancer. Tumors of the female reproductive organs include, but are not limited to endometrial, cervical, ovarian, vaginal, and vulvar cancer, as well as sarcoma of the uterus.

Tumors of the digestive tract include, but are not limited to anal, colon, colorectal, esophageal, gallbladder, gastric, pancreatic, rectal, small intestine, and salivary gland cancers.

Preference is given to colorectal cancer.

Preference is also given to gastrointestinal stromal tumors (GIST).

Tumors of the urinary tract include, but are not limited to bladder, penile, kidney, renal pelvis, ureter, and urethral cancers.

Eye cancers include, but are not limited to intraocular melanoma and retinoblastoma.

Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma.

Preference is given to hepatic cell cancer.

Skin cancers include, but are not limited to squamous cell carcinoma, Kaposi's sarcoma, malignant melanoma, Merkel cell skin cancer, and non-melanoma skin cancer.

Head-and-neck cancers include, but are not limited to laryngeal / hypopharyngeal / nasopharyngeal / oropharyngeal cancer, and lip and oral cavity cancer.

Lymphomas include, but are not limited to AIDS-related lymphoma, non-Hodgkin's lymphoma, cutaneous T-cell lymphoma, Hodgkin's disease, and lymphoma of the central nervous system.

Sarcomas include, but are not limited to sarcoma of the soft tissue, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia.

These disorders have been well characterized in humans, but also exist with a similar etiology in other mammals, and can be treated by administering pharmaceutical compositions of the present invention.

Based upon standard laboratory techniques known to evaluate compounds useful for the treatment of hyper-proliferative disorders, by standard toxicity tests and by standard pharmacological assays for the determination of treatment of the conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The present invention further provides the use of the compound of the invention for the preparation of a pharmaceutical compositions for the treatment of the aforesaid disorders.

### Combination with other pharmaceutical agents:

The compound of the invention can be administered as the sole pharmaceutical agent or in combination with one or more other pharmaceutical agents where the combination causes no unacceptable adverse effects. For example, the compound of the invention can be combined with known anti-hyper-proliferative or other indication agents, and the like, as well as with admixtures and combinations thereof.

Optional anti-hyper-proliferative agents which can be added to the compositions include but are not limited to compounds listed on the cancer chemotherapy drug regimens in the 11th Edition of the Merck Index, (1996), which is hereby incorporated by reference, such as asparaginase, bleomycin, carboplatin, carmustine, chlorambucil, cisplatin, colaspase, cyclophosphamide, cytarabine, dacarbazine, dactinomycin, daunorubicin, doxorubicin (adriamycine), epirubicin, etoposide, 5-fluorouracil, hexamethylmelamine, hydroxyurea, ifosfamide, irinotecan, leucovorin, lomustine, mechlorethamine, 6-mercaptopurine, mesna, methotrexate, mitomycin C, mitoxantrone, prednisolone, prednisone, procarbazine, raloxifen, streptozocin, tamoxifen, thioguanine, topotecan, vinblastine, vincristine, and vindesine.

Other anti-hyper-proliferative agents suitable for use with the compositions of the invention include but are not limited to those compounds acknowledged to be used in the treatment of neoplastic diseases in Goodman and Gilman's The Pharmacological Basis of Therapeutics (Ninth Edition), editor Molinoff et al., publ. by McGraw-Hill, pages 1225-1287, (1996), which is hereby incorporated by reference, such as aminoglutethimide, L-asparaginase, azathioprine, 5-azacytidine cladribine, busulfan, diethylstilbestrol, 2', 2'-difluorodeoxycytidine, docetaxel, erythrohydroxynonyladenine, ethinyl estradiol, 5-fluorodeoxyuridine, 5-fluorodeoxyuridine monophosphate, fludarabine phosphate, fluoxymesterone, flutamide, hydroxyprogesterone caproate, idarubicin, interferon, medroxyprogesterone acetate, megestrol acetate, melphalan, mitotane, paclitaxel, pentostatin, N-phosphonoacetyl-L-aspartate (PALA), plicamycin, semustine, teniposide, testosterone propionate, thiotepa, trimethylmelamine, uridine, and vinorelbine.

Other anti-hyper-proliferative agents suitable for use with the compositions of the invention include but are not limited to other anti-cancer agents such as epothilone and its derivatives, irinotecan, raloxifen and topotecan.

Preference is given to a combination of the pharmaceutical composition according to the present invention and a PD-1/PD-L1(2) inhibitor.

The term "PD-1/PD-L1(2) inhibitor" refers to an anti-PD-1 antibody including but not limited to nivolumab (Opdivo, BMS-936558, MDX1106), pembrolizumab (Keytruda, MK-3475, lambrolizumab), pidilizumab (CT-011), PDR-001, JS001, STI-A1110, AMP-224, tislelizumab, sintilimab, spartalizumab, cemiplimab, dostarlimab, camrelizumab, CS1003 and AMP-514 (MEDI0680), or refers to an anti-PD-Ll antibody including but not limited to atezolizumab (Tecentriq, MPDL3280A), durvalumab (MEDI4736), avelumab (MSB0010718C), BMS-936559 (MDX1105), envafolimab, avelumab, CS1001 and LY3300054 or refers to an anti-PD-L2 antibody.

Preference is given to nivolumab or pembrolizumab or pidilizumab as anti-PD-1 antibody.

Furthermore atezolizumab or durvalumab or avelumab are preferred as anti-PD-Ll antibody.

The term "PD-1/PD-L1(2) inhibitor" also refers to small molecules and peptides including but not limited to BMS-202, BMS-8, BMS-37, caffeoylquinic acid compounds and the GSKa/β inhibitor SB415286.

Most preferred is a combination of combination of the pharmaceutical composition according to the present invention and a PD-1/PD-L1(2) inhibitor wherein the PD-1/PD-L1(2) inhibitor is selected from the group consisting of nivolumab or pembrolizumab.

In case regorafenib is combined with a PD-1/PD-L1(2) inhibitor the daily dose of regorafenib can be reduced. The preferred therapeutic window is a daily dose of regorafenib of a) 55 - 65 mg, preferably 60 mg or of b) 85 - 115 mg, preferably 90 mg, each in combination with a PD-1/PD-L1(2) inhibitor, preferably nivolumab or pembrolizumab, a) or b) depending on the degree of adverse side effects shown in a patient after administration of the combination.

One embodiment of the present invention is a pharmaceutical composition comprising a solid dispersion comprising regorafenib, preferably in amorphous form, wherein the amount of regorafenib in the pharmaceutical composition, preferably a tablet, most preferably an immediate release tablet, is 30 mg.

A further embodiment of the present invention is a pharmaceutical composition comprising a solid dispersion comprising regorafenib, preferably in amorphous form, wherein the amount of regorafenib in the pharmaceutical composition, preferably a tablet, most preferably an immediate release tablet, is 30 mg and the pharmaceutical composition is combined with a PD-1/PD-L1(2) inhibitor. The composition is used for treating cancer, preferably colorectal cancer, hepatocellular cancer, lung cancer (e.g. NSCLC) or gastric cancer.

A further embodiment of the present invention is a pharmaceutical composition comprising a solid dispersion comprising regorafenib, preferably in amorphous form, wherein the amount of regorafenib in the pharmaceutical composition, preferably a tablet, most preferably an immediate release tablet, is 30 mg, and the solid dispersion contains as solid dispersion matrix agent at least one compound from the group consisting of polyvinylpyrrolidone, copovidone, polyethylene glycol and polyethylene oxide or a mixture thereof, which is optionally coated.

One embodiment of the present invention is pharmaceutical composition comprising a solid dispersion comprising regorafenib and at least one pharmaceutically acceptable excipient inside of the solid dispersion, and at least one stabilizing agent outside of the solid dispersion which is selected from the group consisting of methyl cellulose, ethyl cellulose, hydroxyethyl methyl cellulose, hydroxyethyl cellulose, hydroxypropyl methyl cellulose (HPMC), hydroxypropyl cellulose and its acetate, succinate, proprionate, butyrate, adipate, suberate, sebacate and phthalate ester derivatives like carboxy methyl cellulose, cellulose acetate, cellulose acetate phthalate, hydroxypropyl methyl cellulose phthalate, hydroxypropyl methyl cellulose phthalate acetate succinate, hydroxypropyl methyl cellulose acetate succinate and carmellose sodium and mixtures thereof wherein the pharmaceutical composition is enteric coated and the amount of regorafenib is 30 mg and the weight amount of the at least one stabilizing agent in the pharmaceutical composition outside of the solid dispersion is at least 2%, preferably at least 5%, more preferably at least 10%, most preferably at least 15% based on the total weight of regorafenib in the pharmaceutical composition.

Generally, the use of the combinations of the present invention mentioned before will serve to:
(1) yield better efficacy in reducing the growth of a tumor or even eliminate the tumor as compared to administration of either agent alone,
(2) provide for the administration of lesser amounts of the administered chemotherapeutic agents,
(3) provide for a chemotherapeutic treatment that is well tolerated in the patient with fewer deleterious pharmacological complications than observed with single agent chemotherapies and certain other combined therapies,
(4) provide for treating a broader spectrum of different cancer types in mammals, especially humans,
(5) provide for a higher response rate among treated patients,
(6) provide for a longer survival time among treated patients compared to standard chemotherapy treatments,
(7) provide a longer time for tumor progression, and/or
(8) yield efficacy and tolerability results at least as good as those of the agents used alone, compared to known instances where other cancer agent combinations produce antagonistic effects.

"Combination" means for the purposes of the invention not only a dosage form which contains all the components (so-called fixed combinations), and combination packs containing the components separate from one another, but also components which are administered simultaneously or sequentially, as long as they are employed for the prophylaxis or treatment of the same disease.

It should be apparent to one of ordinary skill in the art that changes and modifications can be made to this invention without departing from the spirit or scope of the invention as it is set forth herein.

All publications, applications and patents cited above and below are incorporated herein by reference.

The weight data are, unless stated otherwise, percentages by weight and parts are parts by weight.

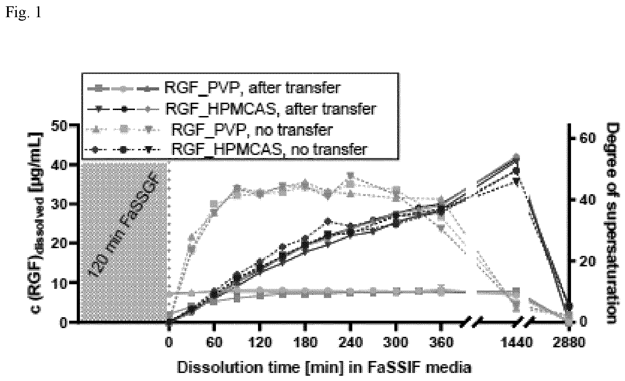

Figure 1: Dissolution in transfer model using gastric (FaSSGF) to intestinal (FaSSIF) transfer for RGF ASDs. Single dissolution curves are shown.

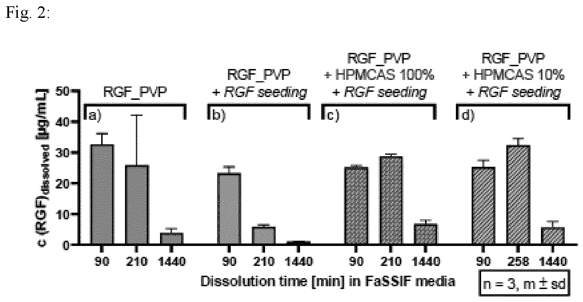

Figure 2: Stabilization of RGF supersaturation of RGF_PVP by addition of HPMCAS in presence of RGF crystal seeds.

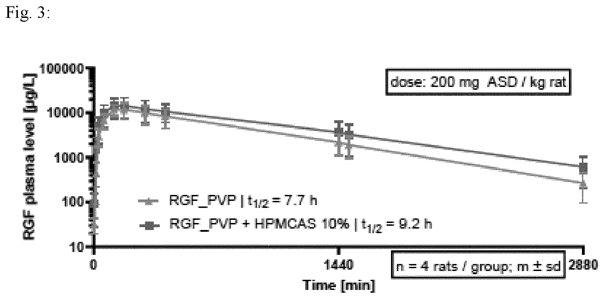

Figure 3: RGF plasma profile in rats after application of RGF_PVP ASD with(out) stabilizer HPMCAS.

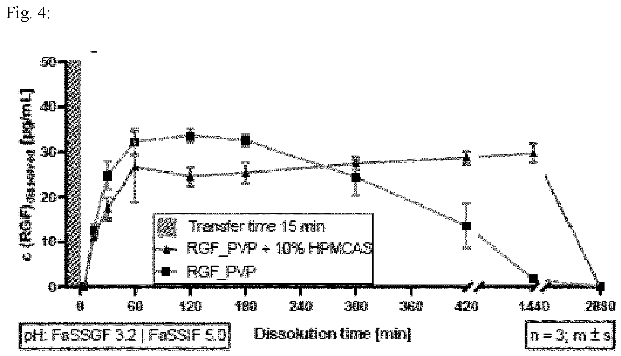

Figure 4: Biorelevant dissolution using transfer model, mimicking rat conditions for RGF_PVP ASD with(out) stabilizer HPMCAS.

### Examples:

### Abbreviations:

ASD - amorphous solid dispersion (containing the active ingredient in amorphous form)
HPMC - hydroxypropyl methyl cellulose
HPMCAS - hydroxypropyl methyl cellulose acetate succinate
PVA - polyvinylalcohol
PVP - polyvinylpyrrolidone
PK - phramakokinetic
RGF - regorafenib
RGF MH - regorafenib monohydrate crystalline
RGF PVP - ASD of regorafenib and PVP
RGF*x*_HPMCAS*y*_PVP*z* - ASD of x parts of regorafenib, y parts of PVP and z parts of HPMCAS
RGF_PVP + *X* %HPMCAS - ASD of RGF_PVP ASD and co-administration of X% by weight of HPMCAS in powder form related to the PVP amount in the matrix
RGF HPMCAS - ASD of regorafenib and HPMCAS

**High Performance Liquid Chromatography (HPLC/UV):** A Dionex P580 system was used, consisting of an ASI-100T autosampler and UVD-340U UV detector with a YMC-Pack Pro C18 RS column. The oven temperature was set to 40 °C and the injection volume was 100.0 µL. UV absorption was measured at 262 nm. Methanol and water with 0.2 % trifluoroacetic acid (TFA) were used as mobile phase in a gradient from 65:35 to 95:5 (v:v) and the flow rate was set to 1 mL/min.

**Biorelevant dissolution studies** were performed in a USP apparatus 2 dissolution vessel at 37 ± 0.5 °C and 75 rpm. To obtain biorelevant conditions, fasted state simulated gastric fluid (FaSSGF) and fasted state simulated intestinal fluid (FaSSIF) were used as dissolution media. For **transfer dissolution experiments**, a dose of 200 mg of the amorphous solid dispersion (ASD) formulations were dissolved in FaSSGF for 120 min prior to FaSSIF equilibration with 500 mL concentrated FaSSIF. **For one-compartment dissolution studies**, only mimicking dissolution at intestinal conditions, the ASD formulations were dissolved in FaSSIF. All samples were filtrated through a 0.2 µm syringe polypropylene filter, considering the API filter adsorption and diluted with methanol for HPLC/UV analysis.

**Biorelevant supersaturation stabilization studies** were performed with an equivalent ratio of ASD dose to FaSSIF media, deviating from the biorelevant dissolution studies method above in total volume of 50-60 mL and in hydrodynamic conditions (ca. 300-400 rpm). The dissolution studies were performed with or without regorafenib monohydrate seeding. For seeding experiments, the same amount of crystalline regorafenib monohydrate was added to the dissolution system as incorporated in the ASD. All samples were filtrated through a 0.2 µm syringe filter and diluted with methanol for HPLC/UV analysis.

Unless otherwise stated, all mean values are calculated from 3 experimental runs. The relative standard deviations (RSD) are calculated from the absolute standard deviations (sd) as RSD = sd / mean ^{∗} 100 %.

### Example 1: ASD containing amorphous regorafenib and PVP and/or HPMCAS and in-vitro dissolution profiles and supersaturation robustness investigations of regorafenib amorphous solid dispersions

### a) Solid dispersion (ASD)

A solution of 6.224 g regorafenib monohydrate (RGF MH, mass calculated to regorafenib) and 24.0 g of PVP (type K25) and/or 24.0 g of HPMCAS (type 716G) in organic solvents was prepared. For an ASD consisting of regorafenib and PVP, a mixture of 85/15 butanone/ethanol (v/v) and for an ASD consisting of regorafenib and HPMCAS, pure acetone was used as solvents. For ASDs consisting of regorafenib, PVP and HPMCAS, methanol was used as solvent. A Rotavapor RII rotary evaporator (Büchi, Essen, Germany) and Variopro PC3001 vacuum pump (Vacuubrand, Wertheim a. M., Germany) were used for solvent evaporation at 60 °C. The resulting ASD was dried for at least 24 h at vacuum and room temperature conditions. The ASD formulations containing PVP as single matrix polymer and the formulations containing both HPMCAS and PVP as matrix polymers were manually ground and sieved to <125 µm mesh size. For ASDs containing HPMCAS as single matrix polymer, grinding was not performed. The investigated formulations are listed in Table 1a.

**Table 1a: Formulation composition of regorafenib amorphous solid dispersion (ASD) in [%] (by weight).**

| Formulation code | Regorafenib (RGF) | PVP | HPMCAS |
|---|---|---|---|
| RGF_PVP | 20 | 80 | - |
| RGF_HPMCAS | 20 | - | 80 |

### Test results:

### 1) Biorelevant dissolution seeding assay

### b) HPMCAS as stabilizer

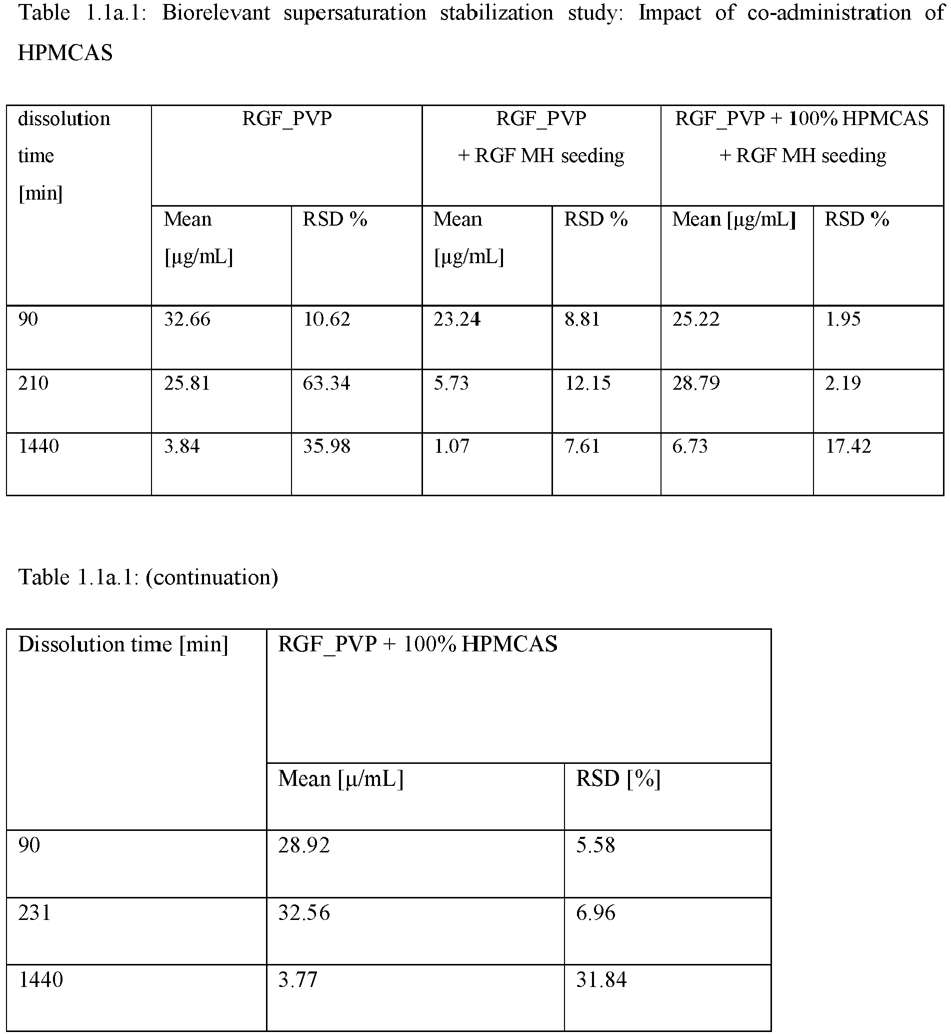

Biorelevant supersaturation stabilization studies with RGF MH seeding were performed as described above.

In Table 1.1a.1, the results demonstrate the impact of regorafenib monohydrate (RGF MH) seeding regarding supersaturation achieved from RGF_PVP formulation and the stabilization properties of co-administered HPMCAS. RGF_PVP leads to fast API release with high supersaturation at 90 min, but is decreased after 210 min. For RGF_PVP+100%HPMCAS, the API release is followed by a more stable supersaturation than in the case of RGF_PVP. Seeding of RGF MH at RGF_PVP dissolution leads to lower dissolved RGF concentrations at these time points. The co-administration of HPMCAS at RGF MH seeding conditions still allows for rapid drug release after 90 min and supersaturation stabilization. HPMCAS co-administration was investigated in a range from X = 5 - 100 % by weight related to the amount of PVP. The results in Table 1.1a.2 show the independence of RGF supersaturation stabilization from the investigated HPMCAS co-administration amounts.

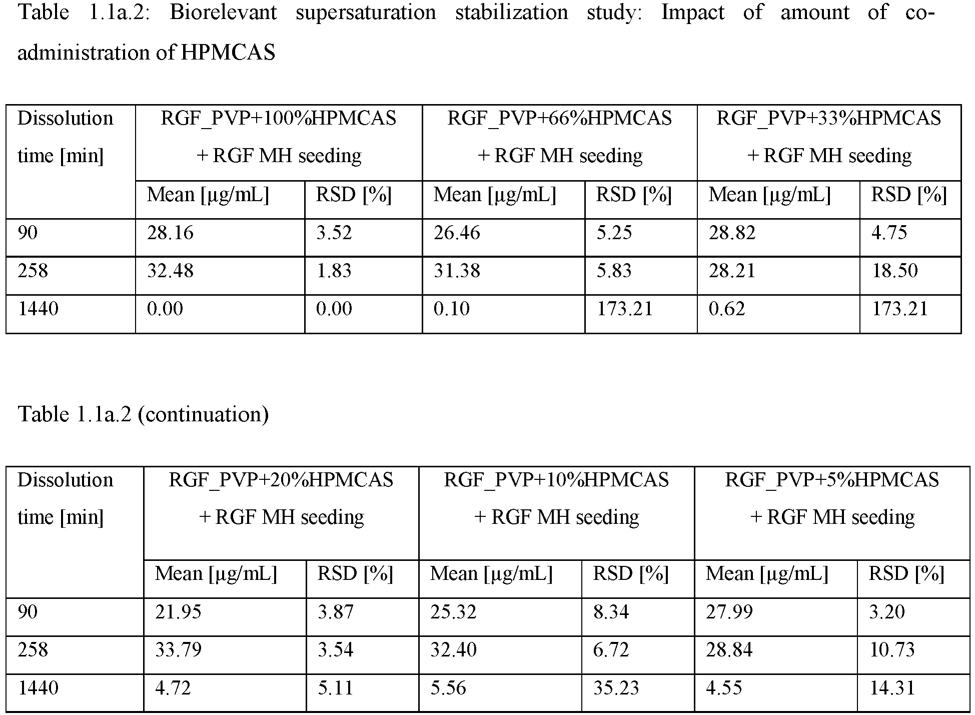

### b) HPMC as stabilizer

Biorelevant supersaturation stabilization studies with RGF MH seeding were performed as described above.

Besides HPMCAS, also HPMC can act as supersaturation stabilizing agent. Methocel E3 Premium LV HP MC (DuPont, Luzern, Switzerland) was used. After 90 min, high RGF supersaturation is achieved, which is stabilized for at least 258 min under seeding conditions, as can be seen in Table 1.1b.

**Table 1.1b: Biorelevant dissolution results of the impact of co-administration of HPMC**

| dissolution time [min] | RGF_PVP + 10% HPMC + RGF MH seeding | |
|---|---|---|
| | Mean [µg/mL] | RSD [%] |
| 90 | 28.68 | 4.37 |
| 258 | 26.05 | 26.34 |
| 1440 | 5.22 | 26.03 |

### c) Biorelevant dissolution: impact of gastric transit

Biorelevant one-compartment dissolution studies were performed for RGF_PVP and RGF_HPMCAS as described above. In Table 1.2.1, our results show the rapid drug release of RGF_PVP, leading to a high supersaturation at 90 min, that is stable for at least 5 h. In contrast, for RGF_HPMCAS less RGF is dissolved for 6 h but the supersaturation obtained is stable for more than 24 h.

Biorelevant transfer dissolution studies were performed for RGF_PVP and RGF_HPMCAS as described above. As shown in Table 1.2.2, RGF_HPMCAS shows slow dissolution to high supersaturation, which is stable for more than 24 h. In contrast, RGF_PVP shows fast dissolution to a reduced supersaturation level, which is reached at 90 min.

These results show the necessity of protection of RGF_PVP from acidic gastric conditions, to allow for both, fast RGF release and prolonged supersaturation.

**Table 1.2.1: Biorelevant one-compartment dissolution studies**

| | RGF_PVP | | RGF_HPMCAS | |
|---|---|---|---|---|
| Dissolution time [min] | Mean [µg/mL] | RSD [%] | Mean [µg/mL] | RSD [%] |
| 0 | 0.00 | 0.0 | 0.00 | 0 |
| 30 | 19.44 | 10.17 | 3.22 | 4.97 |
| 60 | 28.47 | 4.26 | 7.23 | 7.47 |
| 90 | 33.38 | 3.24 | 11.33 | 5.57 |
| 120 | 32.64 | 1.07 | 14.36 | 6.28 |
| 150 | 33.93 | 2.21 | 17.49 | 7.73 |
| 180 | 34.76 | 1.93 | 20.09 | 5.14 |
| 210 | 32.61 | 2.01 | 23.23 | 8.67 |
| 240 | 34.90 | 6.31 | 23.25 | 4.23 |
| 300 | 32.53 | 3.13 | 26.22 | 4.35 |
| 360 | 27.26 | 14.00 | 28.22 | 1.79 |
| 1440 | 4.14 | 14.51 | 37.53 | 4.35 |
| 2880 | 1.54 | 16.92 | 3.98 | 3.41 |

**Table 1.2.2: Biorelevant transfer dissolution studies, including transfer from FaSSGF to FaSSIF conditions after 120 min at FaSSGF conditions**

| | RGF_PVP | | RGF_HPMCAS | |
|---|---|---|---|---|
| Dissolution time [min] after FaSSIF transfer | Mean [µg/mL] | RSD [%] | Mean [µg/mL] | RSD [%] |
| 0 | 5.46 | 55.31 | 0.00 | 0.00 |
| 30 | 6.25 | 32.26 | 2.61 | 9.40 |
| 60 | 7.00 | 21.51 | 6.27 | 3.32 |
| 90 | 7.38 | 14.81 | 9.67 | 5.83 |
| 120 | 7.55 | 10.55 | 13.24 | 4.80 |
| 150 | 7.70 | 6.09 | 15.96 | 5.46 |
| 180 | 7.69 | 5.05 | 18.75 | 4.74 |
| 210 | 7.66 | 6.03 | 20.94 | 5.69 |
| 240 | 7.73 | 2.01 | 23.21 | 4.40 |
| 270 | 7.77 | 3.75 | 24.77 | 6.42 |
| 300 | 7.76 | 2.00 | 26.84 | 4.85 |
| 330 | 7.79 | 1.76 | 28.16 | 3.85 |
| 360 | 7.91 | 4.93 | 29.39 | 2.54 |
| 1440 | 7.32 | 7.84 | 41.50 | 1.44 |
| 2880 | 0.00 | 0.00 | 0.01 | 173.21 |

### d) Ternary ASDs RGF_PVP_HPMCAS dissolution under biorelevant conditions

### a. Biorelevant dissolution at intestinal conditions

To prevent RGF from early precipitation at gastric conditions before entering the small intestine, one approach was embedding RGF in an ASD matrix consisting of two polymers, HPMCAS and PVP, forming ternary ASDs.

Biorelevant supersaturation stabilization without seeding studies were performed as described above, the results are listed in Table 1.3a. For all investigated ternary ASDs, the RGF release rate was decreased in presence of HPMCAS in the ASD matrix.

**Table 1.3a: Dissolution of ternary ASDs RGFx_HPMCASy_PVPz in FaSSIF**

| dissolution time [min] | RGF20_HPMCAS40_PVP40 | | RGF10_HPMCAS45_PVP45 | | RGF20_HPMCAS5_PVP75 | |
|---|---|---|---|---|---|---|
| | Mean [µg/mL] | RSD % | Mean [µg/mL] | RSD % | Mean [µg/mL] | RSD % |
| 90 | 7.47 | 6.31 | 14.46 | 44.68 | 12.46 | 8.17 |
| 258 | 26.78 | 28.92 | 36.83 | 1.69 | 24.91 | 11.47 |
| 1440 | 3.48 | 39.44 | 3.25 | 12.21 | 4.35 | 13.56 |

### b. Biorelevant dissolution at intestinal conditions, including simulated gastric transit

Biorelevant supersaturation stabilization studies without seeding were performed. Deviating from the method described above, a dissolution media transfer was implemented. The ASDs were dissolved in FaSSGF for 120 min. By addition of concentrated FaSSIF media, the conditions were changed to FaSSIF conditions. Results are shown in Table 1.3b.

All investigated ternary ASD formulations showed reduced RGF release after FaSSIF media change, compared to RGF_PVP dissolution at subsection 2. Incorporation of HPMCAS into the ASD matrix does not lead to a rapid RGFG release and long lasting supersaturation.

**Table 1.3b: Biorelevant supersaturation stabilization studies of ternary ASDs RGFx_HPMCASy_PVPz in FaSSIF, after 120 min exposure to FaSSGF conditions**

| dissolution time [min] | RGF20_HPMCAS40_PVP40 | | RGF10_HPMCAS45_PVP45 | | RGF20_HPMCAS5_PVP75 | |
|---|---|---|---|---|---|---|
| | Mean [µg/mL] | RSD % | Mean [µg/mL] | RSD % | Mean [µg/mL] | RSD % |
| 30 | 7.94 | 4.78 | 14.94 | 8.12 | 2.57 | 8.50 |
| 210 | 14.87 | 9.04 | 21.19 | 8.27 | 5.27 | 10.13 |
| 258 | 11.18 | 13.99 | 13.62 | 21.61 | 6.86 | 7.08 |
| 1440 | 5.44 | 2.01 | 6.11 | 16.29 | 4.48 | 2.95 |

### c. Biorelevant dissolution at intestinal conditions, impact of RGF MH seeding

Biorelevant supersaturation stabilization studies with RGF MH seeding were performed as described above. The results from these experiments are given in Table 1.3c. Comparing these results to biorelevant supersaturation stabilization studies without RGF MH seeding, see Table 1.3a, all investigated ternary ASDs lead to robust supersaturation.

**Table 1.3c: Biorelevant seeding dissolution of ternary ASD formulations**

| dissolution time [min] | RGF20_HPMCAS40_PVP40 +RGF MH seeding | | RGF10_HPMCAS45_PVP45 +RGF MH seeding | | RGF20_HPMCAS5_PVP75 +RGF MH seeding | |
|---|---|---|---|---|---|---|
| | Mean [µg/mL] | RSD % | Mean [µg/mL] | RSD % | Mean [µg/mL] | RSD % |
| 90 | 8.30 | 43.82 | 12.03 | 33.68 | 8.68 | 18.58 |
| 258 | 28.98 | 12.11 | 33.63 | 4.73 | 17.69 | 18.70 |
| 1440 | 3.98 | 6.13 | 3.21 | 21.83 | 4.16 | 16.52 |

### e) Biorelevant dissolution mimicking rat conditions

A biorelevant dissolution at rat conditions was performed. The obtained results are in accordance with in-vivo PK data, which are shown in Example 2.

Biorelevant transfer dissolution studies were performed for RGF_PVP and RGF_PVP plus HPMCAS as described above. To mimic the physiological conditions in rats, the pH of FaSSGF was adjusted to 3.2, the pH of FaSSIF was adjusted to 5.0 and transfer time from FaSSGF to FaSSIF was decreased to 15 min.

**Table 4.1: Biorelevant transfer dissolution at simulated rat conditions**

| Dissolution time [min] after FaSSIF transfer | RGF_PVP | | RGF_PVP + 10% HPMCAS | |
|---|---|---|---|---|
| | Mean [µg/mL] | RSD % | Mean [µg/mL] | RSD % |
| 5 | 0.00 | 0.00 | 0.00 | 0.00 |
| 15 | 12.53 | 11.39 | 11.04 | 6.53 |
| 30 | 24.78 | 12.76 | 17.41 | 13.66 |
| 60 | 32.33 | 8.92 | 26.66 | 29.41 |
| 120 | 33.64 | 4.53 | 24.49 | 8.93 |
| 180 | 32.64 | 3.87 | 25.28 | 9.22 |
| 300 | 24.39 | 16.48 | 27.48 | 5.34 |
| 420 | 13.62 | 35.77 | 28.73 | 5.06 |
| 1440 | 1.72 | 11.66 | 29.83 | 7.20 |
| 2880 | 0.00 | 0.00 | 0.00 | 0.00 |

Co-administration of HPMCAS leads to pronounced RGF supersaturation at 420 min and 24 h, as can be seen in Table 4.1.

### Example 2: In-vivo pharmacokinetic (PK) study in rats

ASDs were prepared as described above.

The pharmacokinetic parameters of the compounds according to the invention are determined in male Wistar rats. Oral administration of the drug substance is performed via gavage and the administration volume for rats is 5 ml/kg. The applied dose was calculated to 50 mg RGF / kg rat. After application of the in water pre-suspended ASDs, blood samples were drawn over 48 h and analyzed by LC/MS. The pharmacokinetic parameters are calculated by non-compartmental analysis (NCA). The algorithms for calculating the parameters are defined in an internal process description and are based on rules published in general textbooks of pharmacokinetics.

### Sampling from rat PK study:

Blood samples are removed from the test animals into sodium EDTA (or other anticoagulant)-containing tubes. For sample preparation, 50 µl of plasma are mixed with 250 µl of acetonitrile (the precipitating agent acetonitrile also contains the internal standard ISTD for later analytical determination) and then allowed to stand at room temperature for 5 minutes. The mixture is then centrifuged for up to 8 minutes. The supernatant is taken off, and 500 µl of a buffer suitable for the mobile phase are added. The samples are then examined by LC-MS/MS analysis (e.g. liquid chromatography using a Gemini C18 50 mm x 3 mm column from Phenomenex; by mass spectrometry using an API 6500; SCIEX) to determine the concentration of the test substance in the individual samples.

**Table 2.1: Plasma concentration of regorafenib in rats**

| PK study | RGF_PVP | | RGF_PVP+10%HPMCAS | |
|---|---|---|---|---|
| time [min] | Mean [µg/L] | RSD % | Mean [µg/L] | RSD % |
| 0 | 0 | 0 | 0 | 0 |
| 5 | 31 | 37 | 93 | 70 |
| 15 | 432 | 80 | 1542 | 86 |
| 30 | 2968 | 42 | 5047 | 59 |
| 60 | 6665 | 36 | 9765 | 52 |
| 120 | 11695 | 28 | 14259 | 49 |
| 180 | 11754 | 41 | 14550 | 51 |
| 300 | 9851 | 45 | 12133 | 53 |
| 420 | 8262 | 45 | 10630 | 44 |
| 1440 | 2201 | 50 | 3660 | 69 |
| 1500 | 1915 | 51 | 3272 | 68 |
| 2880 | 269 | 64 | 608 | 67 |

**Table 2.2 Summary: PK study of regorafenib ASDs in rats**

| PK study parameter | RGF_PVP | RGF_PVP+10%HPMCAS |
|---|---|---|
| AUC [kg^{∗}h/L]norm, total | 3.0 ± 41 % | 4.1 ± 54 % |
| t1/2 [h] mean | 7.7 ± 20 % | 9.2 ± 21 % |
| cmax [µg/L] mean | 12346.8 ± 34 % | 14817.3 ± 49 % |

| | | |
|---|---|---|
| AUC = Area under the curve; t1/2 = RGF plasma elimination half time; cmax = maximal observed RGF plasma concentration | | |

Both ASD formulations show plasma variability in rats, results are shown in Table 2.1. For RGF_PVP+10%HPMCAS, AUC, t1/2 and cmax showed superior results as shown in Table 2.2. Biorelevant dissolution experiments mimicking rat conditions predicted a prolonged supersaturation after 420 min (see Example 1, subsection 4), which can be confirmed by the PK data.

### Example 3: Tablet comprising regorafenib and coated with HPMCAS

Coated tablets containing regorafenib were prepared according to the method described in WO 2006/026500. Said tablets were additionally coated with HPMCAS 716G (HPMCAS, Affinisol 716G, Dow Chemical (now DuPont)), as shown in Table 3.1.

### a) Solid dispersion

A solution of 0.415 kg of regorafenib monohydrate (corresponding to 0.40 kg regorafenib) and 1.60 kg of polyvinyl pyrrolidone (PVP 25) in a mixture of 4.80 kg acetone and 1.20 kg ethanol was prepared. Using a fluidized bed vacuum granulator this solution was sprayed onto a powder bed of 1.00 kg croscarmellose sodium and 1.00 kg microcrystalline cellulose at a temperature of 60 - 70°C.

### b) Tableting

The granulate of step a) was roller compacted and screened 3.15 mm and 1.0 mm. Subsequently the compacted granulate was blended with 0.54 kg croscarmellose sodium, 0.0240 kg colloidal anhydrous silica and 0.0360 kg magnesium stearate. This ready-to-press blend was compressed on a rotary tablet press into tablets containing 20 mg and 40 mg of regorafenib.

### c) PVA Film coating

For coating of the 20 mg tablets 0.160 kg of Opadry™ II 85G35294 pink was homogeneously dispersed in 0.640 kg water. For coating of the 40 mg tablets 0.120 kg of Opadry™ II 85G35294 pink was homogeneously dispersed in 0.480 kg water. These coating suspensions were sprayed onto the 20 mg respectively 40 mg tablets of step b) in a perforated drum coater at an outlet air temperature of 35°C. The coating process resulted in evenly coated tablets with a smooth surface. Coating defects could not be observed. Commercially available Opadry™ II 85G35294 pink contains polyvinyl alcohol (partially hydrolyzed) [44% by weight of the total mixture], polyethylenglycol (PEG 3350) [12.4% by weight of the total mixture], lecithin (soya), ferric oxides, titanium dioxide and talc.

### d) HPMCAS Film coating

For the HPMCAS coating of the PVA coated tablets, HPMCAS was dissolved in acetone to a concentration of 6.0 % (m/m). The coating solution was sprayed onto the tablets. A BFC 5 drum coater (Bohle, Ennigerloh, Germany) was used for the coating process, the parameters are given in Table 3.2. Before spraying, the tablets were heated up to an outlet air temperature of 35 °C. After coating, the tablets were dried for 72 h at room temperature to remove residual solvent. The coating process resulted in evenly coated tablets with a smooth surface. Coating defects could not be observed. A total of 20.1 mg of HPMCAS was coated on each tablet.

**Table 3.1: Composition of tablets containing regorafenib**

| Substance | mass [mg/tablet] |
|---|---|
| Regorafenib | 40.00 |
| Polyvinylpyrrolidone (PVP 25) | 160.00 |
| Croscarmellose sodium | 154.00 |
| Microcrystalline cellulose | 100.00 |
| Magnesium stearate | 3.60 |
| Silica colloidal anhydrous | 2.40 |
| HPMCAS | 20.1 |
| Opadry lacquer | 12 |
| Sum | 492.1 |
| Tablet format | oval |
| Dimensions of the tablet | Length: 16 mm, width: 7 mm |

**Table 3.2: Process parameters for HPMCAS coating**

| Parameter | Value |
|---|---|
| Drum speed | 17-19 rpm |
| Air flow | 140-160 Nm³/h |
| Air temperature | 40 °C |
| Atomizer pressure | 0.3 bar |
| Forming pressure | 0.5 bar |
| Spray rate | 32 (initially) - 12 g/min |

### Test results:

The impact of an additional HPMCAS coating on PVA coated regorafenib tablets was investigated by biorelevant dissolution transfer studies as described above. The results obtained are listed in Table 3.3, from each formulation 6 tablets were investigated separately.

**Table 3.3: Biorelevant dissolution transfer studies of Stivarga™ original (Example 3 a) - c)) and HPMCAS coated Stivarga™ tablets (Example 3 a) - d))**

| dissolution time [min] after FaSSIF transfer | Stivarga™ original [µg/mL] | RSD % | HPMCAS coated Stivarga [µg/mL] | RSD % |
|---|---|---|---|---|
| 0 | 28.52 | 15.31 | 0.09 | 244.95 |
| 30 | 29.73 | 19.66 | 12.00 | 11.19 |
| 60 | 27.36 | 22.85 | 23.54 | 4.20 |
| 90 | 25.66 | 28.05 | 28.92 | 4.35 |
| 120 | 23.29 | 34.77 | 31.33 | 4.29 |
| 150 | 21.45 | 38.10 | 33.51 | 6.40 |
| 180 | 19.94 | 41.20 | 33.89 | 2.74 |
| 210 | 18.50 | 41.26 | 35.56 | 2.52 |
| 240 | 17.37 | 43.05 | 36.03 | 2.36 |
| 270 | 16.29 | 41.65 | 36.46 | 1.99 |
| 300 | 15.40 | 41.13 | 36.77 | 3.36 |
| 330 | 14.63 | 36.88 | 36.98 | 2.34 |
| 360 | 14.14 | 36.33 | 37.77 | 2.94 |
| 1440 | 7.50 | 13.81 | 11.50 | 17.45 |
| 2880 | 2.12 | 113.73 | 4.25 | 82.23 |

Stivarga™ original tablets shows supersaturation already directly after media change to FaSSIF conditions, whereas HPMCAS coated Stivarga™ tablets lead to higher RGF supersaturation from 90 min to 1440 min.

To achieve improved bioavailability from poorly soluble regorafenib, the robustness of supersaturation at intestinal conditions is of high importance. This supersaturated state of regorafenib can be stabilized by the polymer. So far, one or more polymers for supersaturation stabilization are incorporated into the ASD matrix. The results demonstrate the superiority of embedding regorafenib in an ASD matrix to be co-administered with the stabilizing polymer HPMCAS or HPMC externally added as supersaturation stabilizing polymers.

## Claims

1. A pharmaceutical composition comprising a solid dispersion comprising regorafenib and at least one pharmaceutically acceptable excipient inside of the solid dispersion, and at least one stabilizing agent wherein the stabilizing agent is outside of the solid dispersion and the pharmaceutical composition is enteric coated.

2. The composition of claim 1 where in the stabilizing agent is selected from the group consisting of methyl cellulose, ethyl cellulose, hydroxyethyl methyl cellulose (HPMC), hydroxypropyl methyl cellulose, hydroxyethyl cellulose, hydroxypropyl cellulose and its acetate, succinate, proprionate, butyrate, adipate, suberate, sebacate and phthalate ester derivatives like carboxy methyl cellulose, cellulose acetate, cellulose acetate phthalate, hydroxypropyl methyl cellulose phthalate, hydroxypropyl methyl cellulose phthalate acetate succinate, hydroxypropyl methyl cellulose acetate succinate and carmellose sodium and mixtures thereof.

3. The composition of any of claims 1 or 2 wherein the stabilizing agent is selected from the group consisting of hydroxypropylmethylcellulose (HPMC), hydroxypropylmethylcellulose acetate succinate (HPMCAS), hydroxypropylmethylcellulose phthalate, hydroxycarboxymethylcellulose, hydroxymethylcellulose, hydroxyethylcellulose, hydroxypropylcellulose, croscarmellose sodium and mixtures thereof.

4. The composition of any of claims 1 to 3 wherein the stabilizing agent is selected from the group consisting of hydroxypropyl methyl cellulose acetate succinate (HPMCAS), hydroxypropyl methyl cellulose (HPMC) or mixtures thereof

5. The composition of any of claims 1 to 4 which is a tablet.

6. The composition of claim 5 is an immediate release tablet.

7. The composition of any of claims 1 to 6 comprising a solid dispersion comprising regorafenib in amorphous form.

8. The composition of any of claims 1 to 7 wherein the solid dispersion comprises a solid dispersion matrix agent selected from the group consisting of polyvinylpyrrolidone, vinylpyrrolidone/vinylacetate copolymer, polyalkylene glycol, hydroxyalkyl, hydroxyalkyl methyl cellulose, carboxymethyl cellulose, sodium carboxymethyl cellulose, ethyl cellulose, polymethacrylates, polyvinyl alcohol, polyvinyl acetate, vinyl alcohol/vinyl acetate copolymer, polyglycolized glycerides, xanthan gum, carrageenan, chitosan, chitin, polydextrin, dextrin, starch, proteins, sucrose, lactose, fructose, maltose, raffinose, sorbitol, lactitol, mannitol, maltitol, erythritol, inositol, trehalose, isomalt, inulin, maltodextrin, β-cyclodextrin, hydroxypropyl-β-cyclodextrin or sulfobutyl ether cyclodextrin or a mixture thereof.

9. The composition of claim 8 wherein the solid dispersion comprises a solid dispersion matrix agent selected from the group consisting of polyvinylpyrrolidone, copovidone, polyethylene glycol, polyethylene oxide or a mixture thereof.

10. The composition of claim 8 or 9 comprising regorafenib and the matrix agent in a weight ratio of 1:0.5 to 1:20.

11. The composition of any of claims 8 to 9 wherein the solid dispersion matrix agent is polyvinylpyrrolidone, croscarmellose sodium and/or microcrystalline cellulose.

12. The composition of any of claims 8 to 9 wherein the pharmaceutically acceptable matrix agent is polyvinylpyrrolidone.

13. The composition of any of claim 12 comprising the regorafenib and the matrix agent in a weight ratio of 1:1 to 1:5.

14. The composition of any of claims 1 to 13 wherein the weight amount of the stabilizing agent in the pharmaceutical composition outside of the solid is at least 2% based on the total weight of regorafenib in the pharmaceutical composition.

15. The composition of claim 14 wherein the weight amount of the stabilizing agent in the pharmaceutical composition outside of the solid is at least 5% based on the total weight of regorafenib in the pharmaceutical composition.

16. The composition of any of claims 1 to 15 wherein the at least one stabilizing agent is present only in the enteric coating.

17. The pharmaceutical composition of any of claims 1 to 16 for use as medicament for treating hyper-proliferative disorders.

18. The pharmaceutical composition of any of claims 1 to 16 for use as medicament wherein the hyper-proliferative disorders are selected from the group consisting of cancers of the breast, respiratory tract, brain, reproductive organs, digestive tract, urinary tract, eye, liver, skin, head and neck, thyroid, parathyroid and their distant metastases.

19. The pharmaceutical composition of any of claims 1 to 16 for use as medicament wherein the hyper-proliferative disorder is hepatic cell cancer or colorectal cancer.

20. A method of treating hyper-proliferative disorders in a subject in need thereof comprising administering effective amounts of the pharmaceutical composition of any of claims 1 to 16.

21. The method of claim 20 wherein the hyper-proliferative disorder is glioblastoma, colorectal cancer, hepatocellular cancer, lung cancer (e.g. NSCLC) or gastric cancer.

22. A pharmaceutical composition comprising a solid dispersion comprising regorafenib wherein the amount of regorafenib is 30 mg.

23. The method of claim 20 wherein the hyper-proliferative disorder is colorectal cancer or hepatocellular cancer.
